# EUROPEAN PATENT SPECIFICATION

(11) **EP 4 202 819 B1**
(45) Date of publication and mention of the grant of the patent: **04.11.2026**
(21) Application number: 22200298.2
(22) Date of filing: 07.10.2022
(51) Int. Cl.: G06Q 30/0601, A61F 9/02, G02C 13/00

(54) **GOGGLE CUSTOMIZATION SYSTEM AND METHODS**
BRILLENANPASSUNGSSYSTEM UND -VERFAHREN
SYSTÈME ET PROCÉDÉS DE PERSONNALISATION DE LUNETTES

(30) Priority: 07.10.2021 US 202163253504 P
(43) Date of publication of application: 28.06.2023
(73) Proprietor: Smith Sport Optics, Inc., Portland, Oregon 97214 (US)
(72) Inventor: LINDAUER, Hans, Brooklyn, New York (US); THORSELL, Eric, Portland, Oregon (US); ROBBINS, Craig, Portland, Oregon (US)
(74) Representative: D Young & Co LLP

(56) References cited:
- CN-U- 213 310 870
- US-A1- 2017 080 172
- US-A1- 2018 017 815
- US-A1- 2019 350 761
- US-A1- 2020 188 175
- US-A1- 2020 409 147

## Description

### CROSS-REFERENCE TO RELATED APPLICATION

This application claims the filing benefit of U.S. Provisional Application No. 63/253,504, filed October 7, 2021.

### FIELD

The present disclosure relates generally to systems and methods for producing form-fitting protective gear customized to a specific user, and more specifically to a goggle customization system and methods.

### BACKGROUND

Goggles are used to protect the user's eyes in various activates. For example snow or ski goggles are often used by users to protect their eyes when participating in various sports, including snow sports, downhill biking and motocross. Goggles may further be used in industrial settings, sometime for use with lenses that are shatter and/or ballistics resistant. Goggles, such as sports goggles, are now frequently designed to have interchangeable lens units to allow the user to easily exchange one type of lens (e.g., a darker tinted lens) for a different lens (e.g., a clear or lighter tinted lens) in order to adapt the same goggle for different use conditions. Goggles with replaceable lenses typically include a goggle frame and one or more removable lenses, which may be part of a lens unit designed for quick and easy lens interchange. The goggle frame may be equipped with a mechanism (e.g., magnetic, mechanical and/or combinations thereof) for removably attaching the lens to the goggle frame. Various advances in the field of goggles has been made but shortcomings remain, for example with respect to providing the desired level of comfort and fit to the user, especially when wearing the goggles for extended periods of time. Thus, further advancements in the field of goggles and similar protective gear may be desirable. US 2018/017815 A1 discloses wearable devices such as eyewear customized to individual wearer parameters. US 2017/080172 A1 discloses systems and methods for making a custom sleep apnea mask or other wearable article. CN 213 310 870 U discloses medical goggles. US 2020/409147 A1 discloses an adjustable electronic device system with facial mapping. US 2020/188175 A1 discloses user-customized goggles.

### BRIEF DESCRIPTION OF THE DRAWINGS

The accompanying drawings, which are incorporated in and constitute a part of the specification, illustrate examples of the disclosure and, together with the general description given above and the detailed description given below, serve to explain the principles of these examples.
FIG. 1 shows an exemplary operational environment for the implementation of the goggle customization solution described herein.
FIGS. 2A and 2B shows examples of a goggle and a goggle frame customizable in accordance with embodiments of the present invention.
FIG. 3 is a flow diagram of a process for customizing a goggle according to the present disclosure.
FIG. 4 is a flow diagram of another process for customizing a goggle according to the present disclosure.
FIGS. 5A-5I show screen captures of a graphical user interface presented on a display of a user device implementing aspects of the goggle customization solution of the present disclosure.
FIG. 6 is a block diagram of a computer-implemented process for customizing a goggle according to the present disclosure.
FIG. 7A shows a visualization of imported face scan data with landmarks
FIG. 7B shows a visualization of the imported face scan data of FIG. 7A, shown here with an example of predefined goggle geometry.
FIG. 8 is a flow diagram of a further computer-implemented process for customizing a goggle according to the present disclosure.
FIGS. 9A and 9B show visualizations, by a CAD program, of face scan data and limiting geometry.
FIG. 10 is a flow diagram of a further computer-implemented process for customizing a goggle according to the present disclosure.
FIG. 11 illustrates a process for fitting one of a plurality of predefined face foams to face scan data in accordance with the present disclosure.
FIG. 12 illustrates an example of distortion analysis as may be performed in the face foam fitting process.
FIG. 13 illustrates an example of a custom generated face flange overlaid onto a user's face scan data.
FIGS. 14A-C show different isometric views of portions of the custom goggle frame.
FIG. 15 shows a computer-implemented process for creating a vent flange lattice in accordance with some examples herein.
FIG. 16 shows another visualization, by a CAD program, of custom-fit portions of the goggle frame overlaid onto a user's face scan data.
FIG. 17 shows another computer-implemented process for generating a custom-fit goggle according to the present disclosure.
FIG. 18 shows examples of product markings onto the custom goggle geometry according to some examples herein.

The description herein will be more fully understood with reference to these figures in which components may not be drawn to scale, and which are presented as various embodiments of the present invention and should not be construed as a complete depiction of the scope of the present disclosure.

### DETAILED DESCRIPTION

A typical goggle includes a goggle frame that supports at least one lens in a spaced apart position in front of the user's eyes to protect the user's eyes (e.g., from debris, sun glare, etc.). The goggle frame encircles the lens and is worn against the user's face, held in place against the user's face by way of a goggle strap coupled to the goggle frame directly, or in some cases via strap outriggers, the strap encircling the user's head to secure the goggle thereto. Because the goggle frame is maintained in contact with the user's face, and often pressed against the user's face for a close fit, the level of fit of the goggle frame is critical to the level of comfort of the user while wearing the goggle. Users' faces come in many different shapes and sizes (e.g., some user's having wider noses, others narrower, others having crooked noses or very pronounced cheekbones, depressed temples, etc.) presenting goggle designers and manufacturer's with a significant challenge in addressing this great variability between the users' faces. While some goggle style may be available in different sizes (e.g., small (S), medium (M), and large (L)), these different standard size cannot fully capture the variability in the shapes/sizes of users' faces and thus cannot provide the level of fit and comfort to all user that may be desirable.

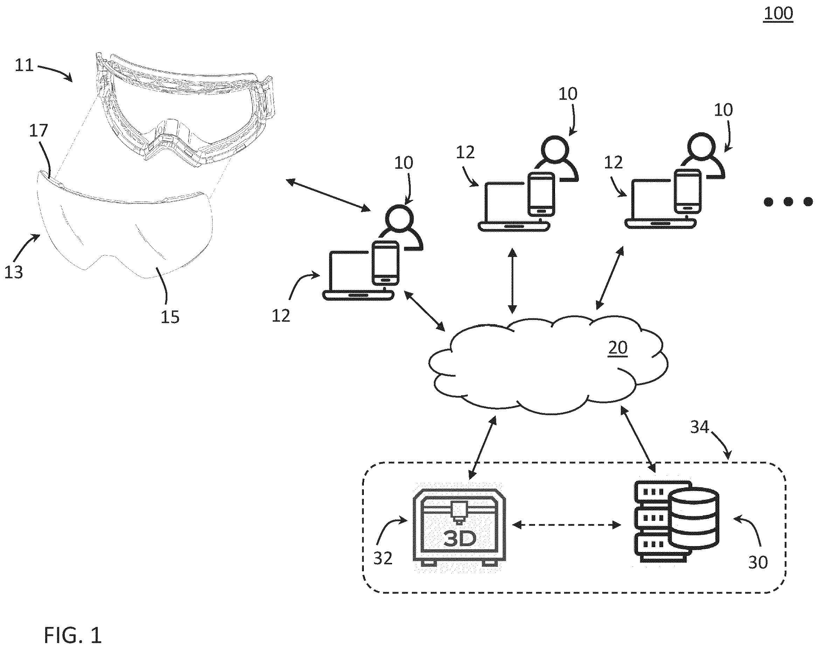

The solution described herein enables producing and providing a user with a truly custom fit goggle, as has not been previously available to consumers. FIG. 1 illustrates an operational environment 100 of the present invention for providing a user with a custom-fit goggle. In a typical operational scenario, there may be at least one user 10 that desires a well-fitting goggle 11. The user may interact with the goggle customization solution via a use device 12. The user device 12 may be any computing device associated with the user, such as a portable computing device (e.g., a laptop, a table, or a mobile communication device (i.e. a smart phone)) or virtually any other computing device that the user has access to. The goggle customization solution may be implemented, at least in part, by software executed by one or more computing devices (or system) 30 associated with the goggle provider (e.g., a goggle manufacturer and/or retailer). In some case, aspects of the goggle customization process, such as the actual production of the customized components (e.g., a custom goggle frame, custom face and/or vent foams, etc.) may be performed by third parties (e.g., a 3D print shop) under the request or specifications from the goggle provider. Custom components may, in some embodiments, be combined or assembled with standard-size components by the goggle provider, or in some cases a third party under the request or specifications of the goggle provider. In some embodiments, components of the goggle customization solution are implemented in a distributed computing system, where one or more components of the software for customizing the goggle resides one or more servers, in some cases on the cloud. The computing device(s) 30 associated with the goggle provider may, thus, also be referred to as server computer(s) or simply server(s), and the user devices 12 may be referred to as client devices. In some embodiments, the computing device(s) 30 include local computing devices included in the distributed computing system, and the goggle customization solution may be implemented by software executed by one or more of the local computing devices.

The computing device(s) 30 and the user device(s) 12 may communicate via a network 20, which can be any suitable communication network including wired and/or wireless components (e.g., a LAN network, a Wi-Fi network, a cellular network, etc.). As will be further described, the goggle customization process outputs one or more custom 3D goggle models, which are provided as 3D-printer compatible files to an additive manufacturing device (also referred to as 3D printer) 32. Various 3D printing technologies, currently known and later developed, may be used to manufacture the custom goggles from the custom 3D goggle models. For example, a custom goggle frame, generated as a result of the goggle customization process described herein, may be printed from a thermoplastic elastomer material, such as thermoplastic polyurethane (TPU), using a multi-jet fusion (MFG) 3D printing technology. Any other suitable 3D printing technology can be used in other embodiments.

In a typical scenario, the user 10 may access a consumer-side application of the goggle customization solution via its user device 12 for performing steps, which will be described further below, to facilitate the customization of the goggle 11. While examples herein will be described with reference primarily to a single user 10, it will be understood that any number of users 10 may interact with the goggle customization solution for obtaining a custom-fit goggle in accordance with the present disclosure. The goggle customization solution may further include a back-end application that receives data provided via the user's interaction with the consumer-side application to generate the custom 3D model(s) of at least one component for the custom goggle. The back-end application is executed by the one or more computing device(s) 30, which may be co-located (e.g., in the same facility 34) with the 3D printer 32 or which may, in some cases, be remotely located and communicatively coupled (e.g., via the network 20). For example, the consumer-side application, the back-end application, components and/or combinations thereof, may be implemented as a Software as a Service (SaaS) solution whereby at least some components reside in the cloud and are accessed (e.g., by the user, or the 3D print shop) via the network 20 (e.g., via the internet).

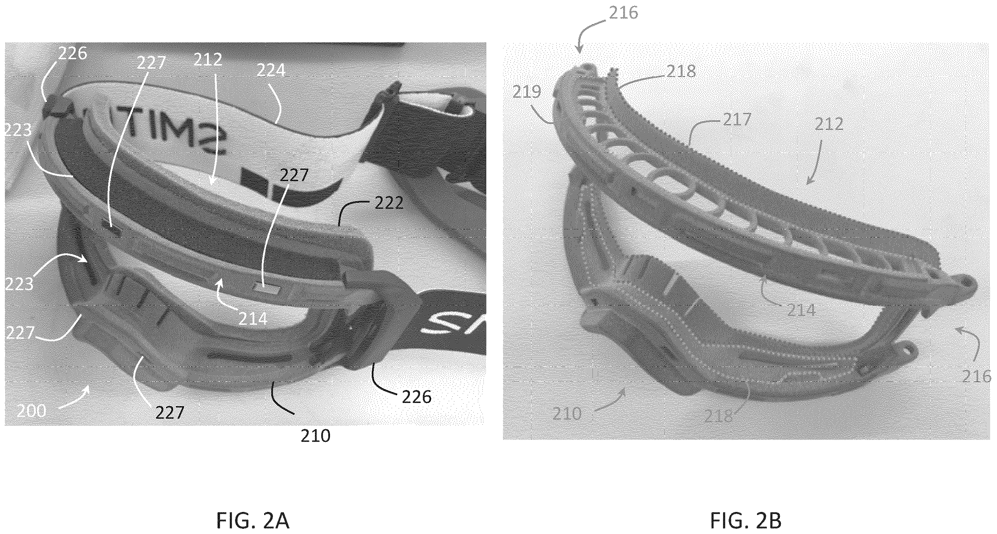

FIGS. 2A and 2B show portions of a goggle. The goggle 200 of FIG. 2A includes a goggle frame 210 having a user-facing side 212, to which a face foam 222 may be attached (e.g., for greater comfort), and a lens-facing side 214. The goggle further includes a strap 224, which is attached, in some cases removably, to the goggle 200, specifically to the lateral sides or ends 216 of the goggle frame 210. The goggle strap 224 may be directly attached to the goggle frame 210, or it may be attached thereto via respective outriggers 226. A lens unit 13 including at least one lens 15 (see FIG. 1) is attached at the lens-facing side 214 of the goggle frame 210. In some cases, the lens 15 is supported on a lens frame 17, forming the lens unit 13. The lens unit 13 may thus include various coupling features (e.g., magnetic and/or mechanical) features for engaging with components of the attachment mechanisms of the goggle that reside on the goggle frame 210 (e.g., magnets 227). In some embodiments, the lens, which may be a single lens or a dual-lens assembly, couples directly to the goggle frame 210, such as through interaction between contouring and/or apertures formed in the lens and the goggle frame 210. Various goggles with removable and/or interchangeable lenses have been developed and are applicable to the examples for customizing goggles described herein. For example, a goggle customizable according to the present disclosure may be any of the goggles described in US Ser. No. 17/202,156, entitled "Goggle with Replaceable Lens", and US Ser. No. 16/672,358, entitled "Goggle Lens with Compound Curvature For Downward Field of View Enhancement,". Of course, the customizable goggle of the present disclosure is not limited to these examples or to goggles with interchangeable lenses. The examples herein may be applied to virtually any goggle currently in the market or later developed. In general it may be desirable to produce a custom-fit goggle while still maintaining the ability to standardize certain components of the goggle, to avoid a cost-prohibitive increase in the production cost and thus consumer price of the goggle. The term standard size or standardize as used to describe the sizing or geometry of components does not necessarily imply a standard across the industry but refers to standard or predetermined sizing/geometry that may be used by a given goggle provider (e.g., in the manufacture and marketing of goggles). Typically, a goggle manufacturer may market a particular goggle type or style in two, three or sometimes four sizes (e.g., Youth, which may also be a Small adult size, Medium size, Large size and sometime an extra-large or XL size) to cover the full range of youth and adult users. Utilizing a limited number of standard or predetermined sizes for a given goggle style is typical and essential to a manufacturer's ability to control manufacturing costs (e.g., tooling costs) and thus the cost of purchase by a consumer. Thus, in some embodiments, it may be advantageous for a custom goggle 200 to include a custom-fit goggle frame 210 having at least one portion (e.g., at the user-facing side 212) that is customized specifically to the shape of the user's face while retaining at least one other portion (e.g., at the lens-facing side) which is not customized but instead utilizes the manufacturer's standard geometry to enable mating of the custom-fit goggle frame with a manufacturer's standard size components, such as a standard size lens unit which may be selected from a predefined plurality of standard size (e.g., a small, medium or large) lens units.

Referring back to FIGS. 2A and now also to 2B, a custom goggle frame 210 may be produced, on demand for any user in accordance with the examples herein. In the custom goggle frame 210, at least some portions of the goggle frame 210 are generated based on the specific user's face shape (or geometry) as will be described further below. For example, the face flange portion, or simply face flange, 217, which is closest to the user's face when the goggle is worn, and the vent flange portions, or simply vent flanges, 218 that connect the face flange 217 to the lens-side portion 219 of the goggle, are customized through the process described herein, based upon the user's face geometry. In some embodiments, the lens-side portion 219, at the lens-facing side 214 of goggle frame 210, is of standard geometry (e.g., pre-specified by the goggle provider prior to the customization process). In some embodiments, other components of the goggle frame 210, such as the outriggers 226 and/or nose piece, if present, may be of standard geometry. In some embodiments, the one or more foams (e.g., face foam 222, vent foams 223) may also be of standard geometry. In some embodiments, components of the goggle frame may be selected from a set of standard geometries. For example, the lens-facing side 214 of goggle frame 210 may have two or more different standard geometries that are selected based on the customization of the goggle and the user's face geometry. Other components of the goggle frame may be selected from two or different standard geometries as well. In other embodiments, it is envisioned that at least some of the above "standard" components, such as one or more of the foams and/or the nose piece, are also customizable. In some embodiments of the present disclosure, the entire goggle frame 210, excluding any standard size components such as outriggers, nose piece, and lens attachment components/features (e.g., magnets 227), if present, is produced as single, unitary body, using a 3D printing process. Other customized components of the goggle, e.g., custom face foam(s), custom nose piece, etc., may also be produced from a custom geometry/model of that component. For example, custom foams may be laser cut from the custom geometry, and custom components of higher rigidity than the goggle frame may be 3D printed from a suitable polymer (e.g., nylon, ABS or PLA) using any suitable 3D printing technology (e.g., stereolithography (SLA), fused filament fabrication (FFM), multi-jet fusion (MJF) or others). To assemble the goggle into its final configuration for delivery to the user, components such as the outriggers, nose piece, and lens attachment features are assembled to the goggle frame. The face foam and vent foams, which may be of standard sizes or custom, are also assembled (e.g., glued) to the goggle frame and the strap and lens are added to provide a finished product, ready for use by the user.

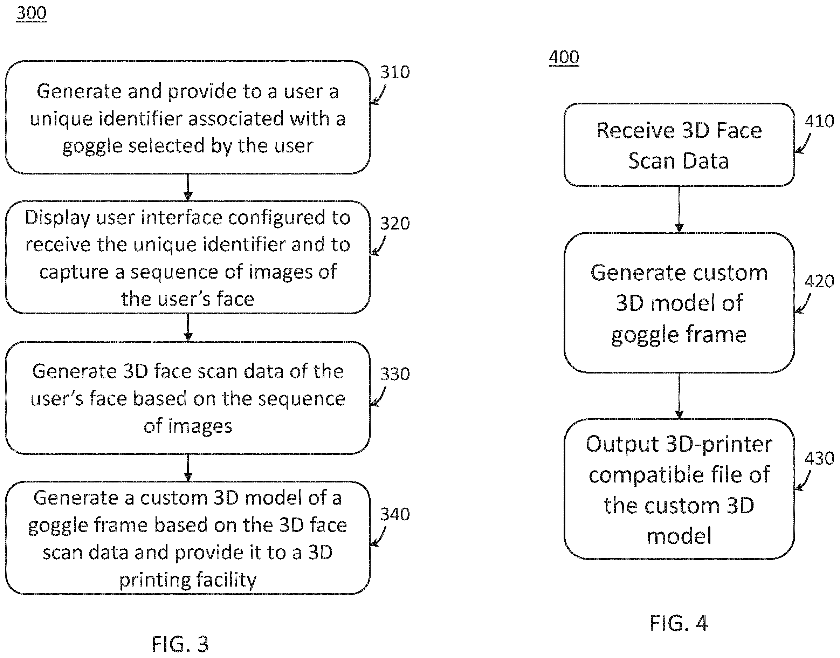

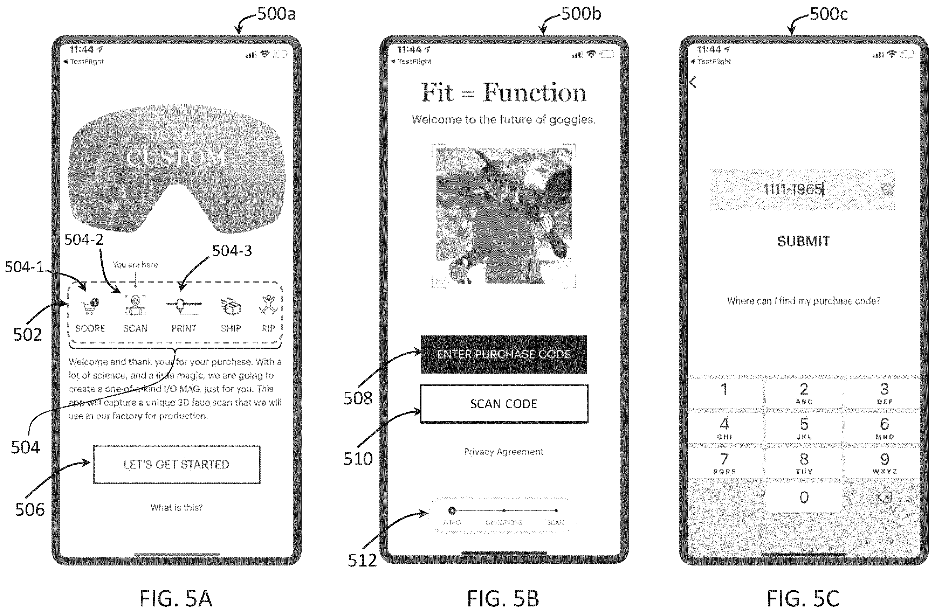

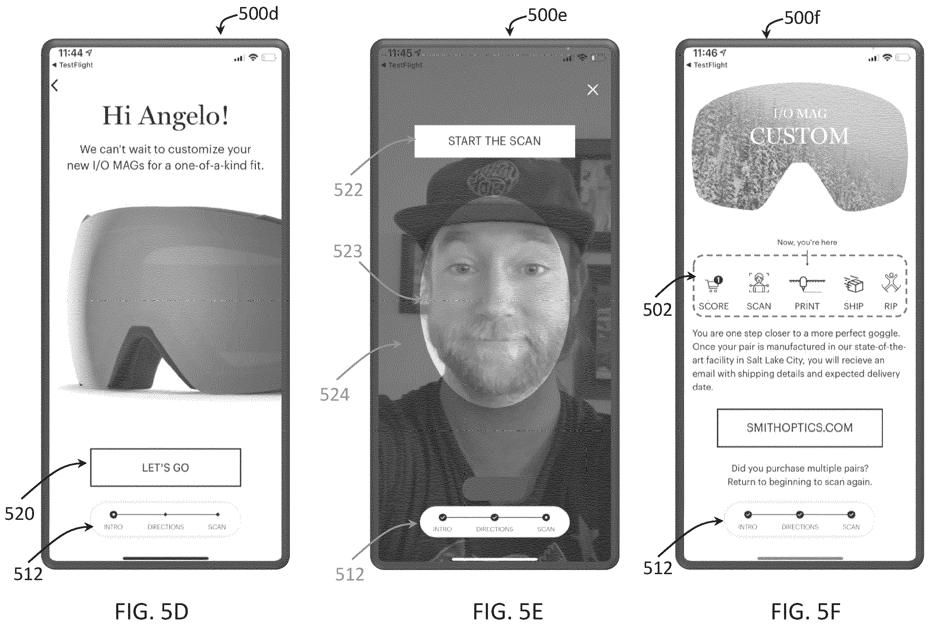

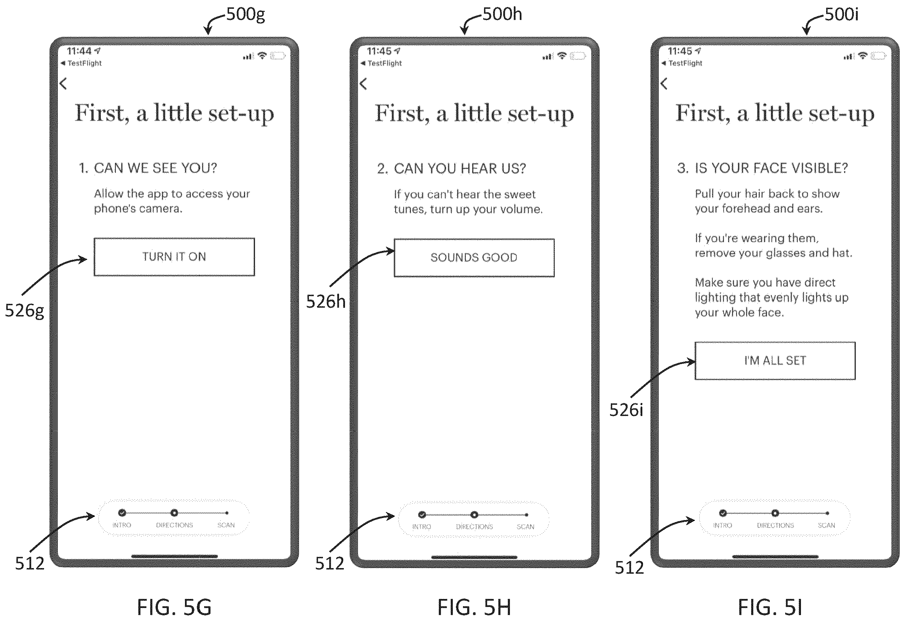

FIGS. 3 and 4 show block diagrams of process 300 implemented, at least in part, by the user device 12, and process 400 implemented by the computing device 30, respectively. The process 300 in FIG. 3 will be described in further detail with reference also to FIGS 5A-5I, which show screen captures of an example graphical user interface that may be presented on the user device 12 when executing the consumer-side application, also referred to as the user app, of the goggle customization solution. Process 300 may start with the generating and providing to a user 10 of a unique identifier, as shown in block 310. The unique identifier may be associated with a goggle selection whereby the user selects a goggle for customization. For example, the user may purchase a goggle, e.g., via the goggle provider's e-commerce web-site or via a brick-and-mortar retail store and may receive the unique identifier upon completion of the purchase. The unique identifier, which may also be referred to herein as the user's purchase code, goggle customization code or simply project code, may take any suitable form. For example, the unique identifier may be provided to the user as a text string (e.g., comprising a combination or string of alpha numeric characters), or as an electronically-readable code (e.g., a Bar code, a QR code, or the like).

Referring back to FIG. 3, the process 300 continues with step 320 in which a user interface is presented to the user for receiving the unique identifier and for capturing data about the user's face, or portion of the user's face (e.g., portions including pupils, eye sockets, lips, nose bridge, temples, brows, forehead, cheeks, chin, or any combination of the features). Such user interface(s) may be presented to the user via the user app, through which the user 10 initiates, and interacts with, the goggle customization solution. The user 10 may be presented with a welcome screen, for example as shown in the screen capture 500a of FIG. 5A. The welcome screen (e.g., screen capture 500a) may include a customization process graphic 502, designed to inform the user about the various steps 504 of the goggle customization process (e.g., goggle selection/purchase step 504-1, face scan step 504-2, custom build step 504-3, etc.). The user interface of the welcome screen may enable the user to imitate the customization process, e.g., via a first start button 506. In some embodiments, the welcome screen is omitted and upon launching the user app, the user is presented with one, or a sequence of, user interface screen(s) that enable the user 10 to submit their project code (see e.g., screen captures 500b and 500c of FIGS. 5B and 5C), which may also be referred to as "code submission" screen(s). In the illustrated example, the user app is configured to enable the user to select the mode for submitting the project code. To that end, the user app presents a first code submission screen (e.g., as shown in screen capture 500b of FIG. 5B). The first code submission screen includes a plurality of code input buttons (e.g., first and second code input buttons) 508 and 510 each of which is associated with a different mode of user input for submitting the project code. A first code input button 508 may be configured to enable the user to select manual submission and may activate a second code submission screen 500c that enables the user to input the code manually, e.g., via a keyboard or number pad presented on the second code submission screen 500c. A second code input button 510 of the first code submission screen 500b may activate the user device's camera or other scanning device connected to the user's device, for scanning the electronically-readable code. Other modes for inputting the project codes are envisioned, for example via voice command. In such scenarios, a code input button may be presented on the first code submission screen, which, upon selection, activates the user device's microphone.

Upon receipt of the user input (e.g., the unique identifier), the user app may display a face scan initiation screen (e.g., as shown in screen capture 500d of FIG. D). In some embodiments, it is envisioned that the user's unique identifier (or project code) may be transmitted directly to the user app (e.g., from the goggle provider's e-commerce platform) and the user may receive a notification or reminder (e.g., via the user app and/or via a direct message - e-mail or text) to complete the goggle customization process. In some such scenarios, the user app may present a face scan initiation screen upon launching of the user app, omitting at least one of the previously described interface screens. In other embodiments, additional interface screens may be presented before or at this point of the user's customization journey, as will be further described below.

The face scan initiation screen (e.g., screen capture 500d) may include a second start button 520, upon the selection of which the user app may display a face scan screen (e.g., as shown in the screen capture 500e) to enable the user to collect data about the user's face, or a portion of the user's face. Upon selection of the second start button 520, the user app may also activate a sensor included in the user's device to collect the data about the user's face. For example, in some embodiments, a user device's camera is activated to collect a sequence of images of the user's face, particularly a camera positioned to face the user (e.g., a camera on the display side of the device 12). In some embodiments, a sensor such as a LIDAR scanner is activated to collect data about the user's face (e.g., voxels, point cloud data, etc.). The activation of a sensor may be automatic, upon the pressing of the second start button 520, or it may be responsive to the user selecting the appropriate sensor (e.g., selecting a camera-rotate button on a smart phone). The face scan screen (e.g., screen capture 500d) may be configured to display a live feed 523 from the user's camera, which with a user-facing camera may display the user's face, as shown in FIG. 5E. In some embodiments, a mask 524 may be applied in the face scan screen such as to provide feedback to the user on properly positioning/orienting their face with respect to the user device/camera. In some embodiments, the face scan screen may initiate the recording of the data for the user's face automatically, e.g., upon determining that the user's face is suitably positioned with respect to the sensor. In some embodiments, the user device 12 may guide the user (e.g., via audible commands and/or messages overlaid onto the face scan screen) through the capture process. In some embodiments, the face scan screen may include a third start button 522 configured to initiate the face scan (e.g., the recording of images of the user's face).

Referring back to FIG. 3, the process continues with step 330 in which 3D face scan data of the user's face generated based on the data collected via sensors of the user device. For example, when the data about a user's face includes a sequence of images, the 3D face scan data may be generated from the sequence of images using any suitable technique. For example, the 3D face scan data may be generated in accordance with the examples described in US Pat. App. Pub. No. 2020/0105013, entitled "Robust Head Pose Estimation with Depth Camera,". The 3D face scan data may be generated using any other suitable algorithm that can produce a 3D data set representing the shape (e.g., geometry) of the user's face. The 3D face scan data may be generated by the user device 12, or by another computing device (e.g., the computing device 30) communicatively connected to the user device for receiving the data about the user's face (e.g., sequence of images, voxel data, point cloud data). Once the 3D face scan data has been generated, and referring back to FIG. 3, the process 300 proceeds to step 340 in which a custom 3D model of a goggle frame is generated based, at least in part, on the 3D face scan data. As will be further described, the custom 3D model may be generated in accordance with any of the examples herein, e.g., by the computing device(s) 30. The custom model is subsequently provided, in a suitable file format to the 3D printer 32, which as previously noted may be co-located (e.g., at the same building or in a same manufacturing plant or facility 34), or which may be remotely located and communicated to the 3D printer, e.g., via the network 20. Continuing with the user experience example (of FIGS. 5A-5F), and upon completion of the image collection and 3D face scan data generation, the user app may display a completion screen, e.g., as shown by screen capture 500f of FIG. 5F. The completion screen may include at last one process timeline graphic. For example, the completion screen may include the customization process graphic 502, updated to show the process steps completed through the user's interaction with the user app. Additionally or alternatively, the completion screen and/or any of the preceding screens displayed during the user's journey with the app may include a face scan process graphic 512, which is updated as the user advances through the journey (e.g., from interface screen 500b through the completion screen) to provide feedback to the user as to the user's progress through the customization process.

In some embodiments, the user experience may involve additional, optional steps, where the user app presents additional, optional screens to guide the user at various stages of the user journey. For example, the user app may present one or more set-up screen 500g, 500h, and 500i, to aid the user and/or receive confirmation of the user's device being suitably configured to perform the data collection process. One or more of the set-up screen 500g, 500h, and/or 500i may be presented to the user, e.g., in a sequence or interspersed with other screen at appropriate times. In one embodiment, the one or more set-up screens 500g, 500h, and/or 500i are presented between the face scan initiation screen and the face scan screen. For example, one or more of the set-up screen 500g, 500h, and/or 500i may be invoked by the pressing of the second start button 520 instead of invoking the face scan screen 500e. In some such embodiments, the face scan screen 500e may be invoked upon appropriate user input via the one or more set-up screens 500g, 500h, and/or 500i. In some embodiments, the one or more set-up screens 500g, 500h, and/or 500i, are invoked upon selection of optional set up button on the face scan initiation screen (e.g., screen 500d). In other embodiments, the one or more set-up screens 500g, 500h, and/or 500i are presented following the welcome screen, such as upon selection of the first start button. In yet other embodiments, at least some of the set-up screens, e.g., screen 500h, may be presented at a one time during the user journey, e.g., earlier such as after the welcome screen, while other ones of the set-up screens (e.g., screen 500g and/or 500i) are presented at another time during the use journey, e.g., at a later time such as before initiating the face scan. The one or more set-up screen 500g, 500h, and/or 500i may instructor query the user with respect to the set-up of the user device and may enable the user to advance through the user journey upon receiving appropriate user input (e.g., configuration via the respective button 526g, 526h and 526i).

Referring back to FIG. 4, the computing device 30 associated with the goggle provider may implement a server-side (or back-end) application associated with the goggle customization solution, and may thus be referred to as server, which does not necessarily imply a particular deployment of the back-end application. In some embodiments, the computing device 30 implements a process involving the steps of receiving 3D face scan data of a user (block 410), generating one or more custom 3D models (e.g., a custom 3D model of a goggle frame) based, at least in part, on the 3D face scan data (block 420), and outputting the custom 3D model(s) in a 3D-printer compatible file format (block 430), which may subsequently be provided, for example to a 3D printer, to produce the custom-fit component(s) of the goggle. The 3D face scan data of the user may be stored by a server of the computing device 30, and the 3D face scan data of the user is imported to a local computing device of the computing device 30 for generating one or more custom 3D models based, at least in part, on the 3D face scan data, and outputting the custom 3D model(s) in a 3D-printer compatible file format (block 430).

In some embodiments, a computer-implemented method, which may be performed by the computing device 30, includes, importing a first three-dimensional (3D) data set that represents the user's face or a portion of the user's face (e.g., it's 3D shape). The computer-implemented method may further include importing a second 3D data set comprising predefined goggle geometry. The predefined goggle geometry includes predefined geometry of at least a lens-side portion of a goggle frame. The computer-implemented method further includes creating a custom goggle frame model based, in part, on the first and second 3D data sets. The creating of the custom goggle frame model includes, creating custom geometry of a first portion of the goggle frame proximate the user's face based, in part, on the first 3D data set, and creating custom geometry of a second portion of the goggle frame by connecting the custom geometry of the first portion to the predefined geometry of the lens-side portion of the goggle frame. The custom model generated by this process may be exportable as a 3D-printer compatible file. In some embodiments, the first 3D data set and the second 3D data set are imported into a CAD modeling environment, and the custom model is exportable from the modeling environment as a 3D-printer compatible file.

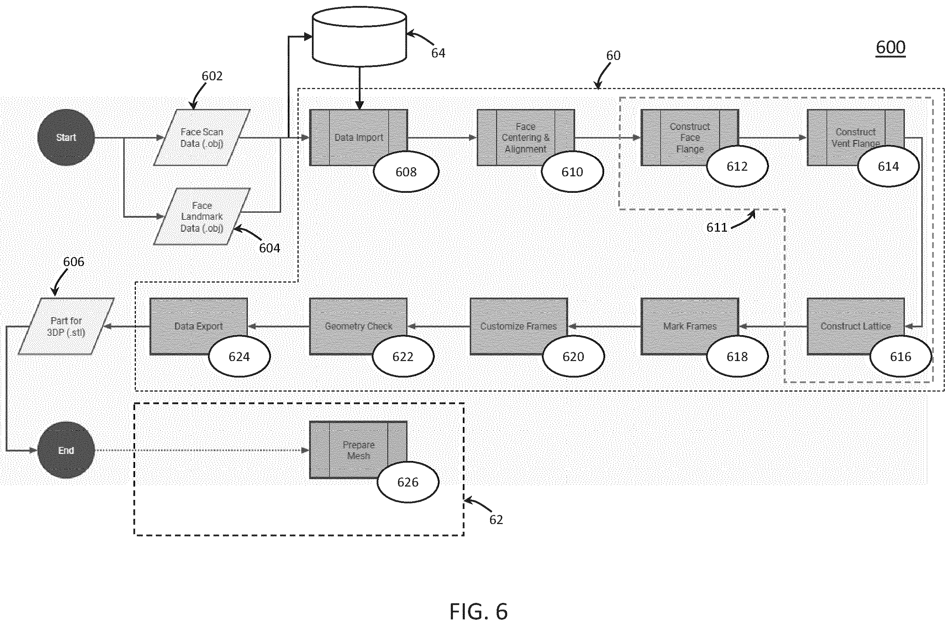

FIG. 6 shows a flow diagram of an example process 600, which may be implemented by a processor 60 of the computing system 30. While referring here to a processor, it will be understood that the functions of any processor described herein, such as processor 60, may be performed a single processing unit (e.g., a CPU or GPU) or by multiple processing units arranged to operate in distributed manner (e.g., in networked computers, or arranged for parallel processing). The processor 60 receives a 3D dataset that represents the shape of the user's face or a portion of the user's face. This dataset, which is referred to herein as 3D face scan data (see block 602), may be obtained through scanning of the user's face or any other suitable means. The 3D face scan data may thus include a 3D representation of the user's face, and may be provided to the computing system 30 in any suitable file format, such as but not limited to an OBJ (.obj) file, an STL (.stl) file, a STEP (.stp or .step) file, and IGES (.igs) file, etc., that is used for storing geometry of a 3D object (i.e. 3D data). In some embodiments, the 3D representation of the user's face (or at least a portion of the user's face) may include a 3D mesh representation of the user's face. The 3D representation of the user's face may alternatively or additionally include other 3D information about the user's face in some embodiments.

In some embodiments, the face scan data 602 may be accompanied by face landmark data 604 which identifies landmarks in the 3D geometry contained in the face scan data. The face landmark data 604 may be similarly provided to the computing system 30 (e.g., processor 30) using any suitable file format for storing 3D geometry (e.g., mesh) data (e.g., an OBJ file, STL file, or other). In some embodiments, the 3D face scan data 302 and the optional face landmark data 604, collectively user (or face) geometry data, may be provided to the computing system 30 (e.g., to processor 60) directly by the user device 12. In some embodiments, the user geometry data may be stored in a storage device 64 before they are received by the processor 60. The storage device 64 may be a local storage device associated with processor 60. In some embodiments, the storage device 64 may include remote (e.g., distributed, networked) storage. In some embodiments, multiple datasets of user geometry data, associated with different users and/or different customization projects, are stored (e.g., in storage 64) and queued up for ingestion into the goggle customization tool.

Upon receiving the user geometry data (either from the user device directly or retrieved from storage 64), the processor 60 may import the user geometry (see block 608) into a 3D modeling environment of a computer-aided design (CAD) program, which may be executed (in the foreground or background) by processor 60 as part of the goggle customization process. Importing the data (e.g., at block 608) may involve querying a first database containing all queued customization projects to identify any not completed customization projects. A variety of CAD modeling programs exist for use in defining (or modeling) 3D geometry of object(s) (e.g., during product development), including but not limited to AutoCAD (provided by Autodesk), SOLIDWORKS and CATIA (provided by Dessault Systemes), Solid Edge (provided by Siemens), Rhino (developed by Robert McNeel & Associates), Creo (developed by Parametric Technology Corporation), etc. Any of these or any other suitable CAD modeling program may be used in embodiments of the present disclosure. Depending on the CAD modeling program used, the importing of the user geometry data (at block 608), and optionally the predefined geometry, may involve converting the data into a format specific (or native) to that CAD program, which may or may not be compatible with other tools (such as other CAD programs or the 3D printer). In addition to the user geometry data (e.g., face scan data and landmark data), the processor 60 may access (e.g., retrieve from storage 64) and import the predefined (or predetermined) goggle geometry into the same 3D modeling environment that contains the user-specific geometry. The order of importing the geometry is not material. In some cases, the face scan data is imported first, followed by the predefined geometry. In other cases, this order is reversed. In some cases, the predefined goggle geometry may be imported at a later time and/or in parts, such as by importing a first portion of the predefined geometry (e.g., a first set of curves, surface, or components of the goggle) prior to the preparation (e.g., alignment, trimming, mesh coarseness adjustments) of the user-specific geometry, and importing a second portion of the predefined geometry (e.g., certain other curves, surfaces or components of the goggle frame) after the preparation of the user-specific geometry. In some embodiments, at least some of the preparation of the user-specific geometry (e.g., global alignment) may be performed, for example relative to a coordinate system of the D modeling environment, prior to importing the predefined geometry.

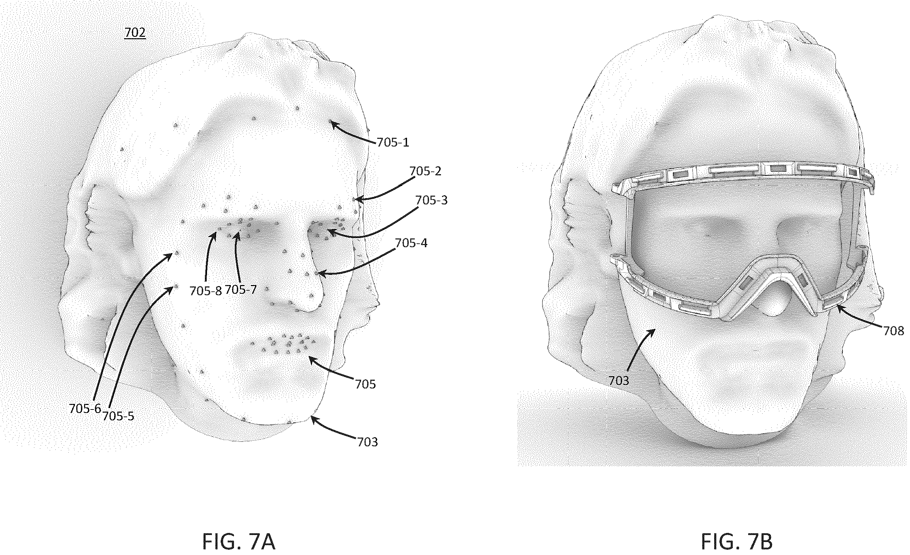

FIGS. 7A and 7B show example visualizations (e.g. as generated within a CAD modeling program) of user geometry data 702 and predefined goggle geometry 706. The user geometry data 702 (which in some embodiments may be obtained from the images acquired of the user's face, such as previously described with reference to FIGS. 3 and 5A-5I) may include 3D face scan data 703 which represents the shape of the user's face. The face scan data 703 is visualized in FIG. 7A as a surface(s) rendering. The user geometry data 702 may also include face landmark data 705 comprising discrete landmarks (or data points) of certain anatomical features of the user's face (e.g., pupils, eye sockets, lips, one or more points defining features of the nose such as center, bridge, width, or other, one or more points defining the temples, brows, forehead, cheeks, chin, etc.). In some embodiments herein, not all data points of the face landmark data 705 is used during the goggle customization. For example, and as discussed further below, only one or a subset of the face landmark data 705 (e.g., the pupils, and in some cases forehead, brow and/or nose land mark data points) may be used for the customization. In FIG. 7A, the landmark data 705 is shown overlaid with the face scan data 703 for illustration. In some cases, the face scan data 703 and/or the landmark data 705 may be imported, manipulated and/or visualized separately from the other. The 3D face scan data may additionally or alternatively be visualized by the underlying 3D/polygon mesh (without any surface shading).

In FIG. 7B, the face scan data 703 (visualized here again as rendered surfaces) is shown together with a rendering of predefined goggle geometry 708. The predefined goggle geometry 708 may differ in different embodiments. In some embodiments, the predefined geometry 708 includes geometry defining at least a portion of the goggle frame (e.g., the lens-side portion 219. In some embodiments, the predefined geometry 708 includes, additionally or alternatively, geometry of components of the goggle that are formed separately from the goggle frame, for example geometry of the strap outriggers, magnetic and/or mechanical latch components, a nose piece, one or more of the face foams of the goggle, or any combinations thereof. In some embodiments, certain additional components of the goggle (e.g., one or more of the face foams) which are formed (or manufactured) separately from the goggle frame may also be associated with custom geometry and thus additional custom goggle geometry is defined, as part of the customization process to enable the manufacturing (e.g., laser-cutting) of these additional custom components. In some embodiments, the predefined geometry 708 includes, additionally or alternatively, only partial definitions of the above such as certain curves or surfaces of the lens-side portion 219, the outriggers, the nose piece or foams, but not all curves and surfaces needed to fully defined these 3D objects. For example, the predefined geometry 708 may include a portion of the geometry defining the outriggers or nose piece, such as the curves or surfaces on the user-facing side of the goggle, while curves or surfaces on the lens side of the goggle are omitted. In some examples, the predefined geometry 708 used in a given goggle customization project may be selected based, in part, on user inputs. For example, in some cases, the user may select a size from a predetermined number of available sizes (e.g., Small, Medium or Large) at the time of goggle selection (e.g., prior to step 310 of the process 300), and the user's size selection may be associated with the user's unique project identifier, such that the information about the user-selected goggle attribute is accessible to the customization system 30 (e.g., processor 60). The processor 60 can then select from among a plurality of available predefined goggle geometries, each associated with a different user-selectable attribute, for import and use in the goggle customization process. Other attributes selectable by the user may include size, color, texture, or material of certain components (e.g., the foams, the outriggers, nose piece, strap, etc.). One or more, and in some cases all, user-selectable attributes may be used for generating the custom goggle geometry and/or for generating a custom marker on the goggle frame, as will be further described below.

Referring back to FIG. 6, in some embodiments, the user geometry data 702 is processed (at block 610), as will be further described below, to prepare it for use in creating the custom goggle geometry (e.g., in blocks 611) and generating the one or more custom 3D models. In some embodiments, the creating of custom geometry may involve the construction of a face flange (block 612), the construction of one or more vent flanges (block 614), and the defining of a lattice in one or more of the vent flanges (block 616). In some embodiments, the creating of custom geometry may further involve the fitting of a face form of a selected predetermined size prior to creating the face flange. In other embodiments, the creating of custom geometry may further involve the creation of the custom face foam geometry. As will be further discussed below the one or more custom 3D models may include a combination of predefined goggle geometry 708 and custom goggle geometry that is unique and specifically created in the model for use for the particular user and/or based on the user's particular face geometry (e.g., based on the face scan data 703). In some embodiments, process 600 may further include one or more geometry validation check(s) (block 622). Geometry validation may be performed throughout the geometry manipulation (e.g., alignment) and/or creation processes to ensure optimal custom-fit for the user. It should be noted that the difference between an optimal fit or discomfort may be the result of very small differences in geometry (e.g., one or two degrees of misalignment). Geometry validation may be performed upon the creation of the custom 3D model(s) such as to confirm that the model created by the customization process is suitable for 3D printing (e.g., there are no gaps in portions of the model that should define solid geometry, etc.). Finally, the custom 3D model(s) for the custom-fit goggle may be exported (at block 624) as a 3D-printer compatible file 606. The exporting step 624 may in effect be the reverse of the importing step 608, whereby the geometry defined within the CAD modeling program, which may be a format unique to the CAD modeling program, is converted to a portable (agnostic to a specific CAD software) 3D file format such as, but not limited to, OBJ, STL, STEP, IGES, etc. The custom 3D model of the custom-fit goggle may then be provided to a 3D printer 34 to produce the custom-fit goggle. In some embodiments, the 3D geometry in the 3D-printer compatible file 606 may be further processed (see block 626) at the printer 34 (e.g. by a processor 62 of the printer 34) to present the geometry data to the printer 34 in a manner (e.g., in slices or layers of the 3D object) suitable for the particular 3D printing technology used.

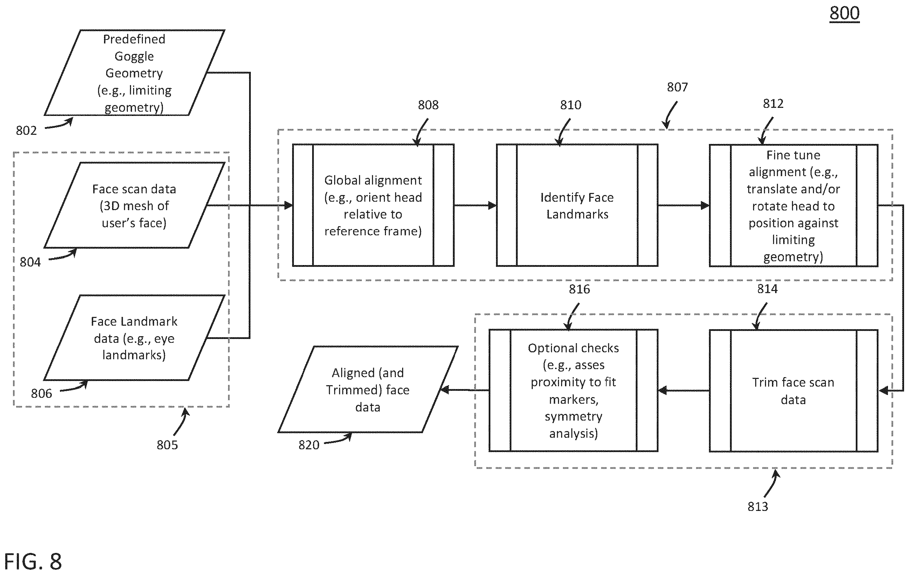

FIG. 8 shows an alignment process 800 according to some examples of the present disclosure. One or more of the blocks of process 800 may be used to implement the face centering and alignment block 610 of process 600. The blocks of process 800 may be executed in different order, and/or one or more of the blocks of process 800 may be omitted or substituted by other suitable blocks in other embodiments. Process 800 receives as input the user-specific data 805, which may include the user's face scan data 804 and landmark data (or a portion thereof) 806, e.g., as imported at block 608 of process 800. The process 800 may further receive as input the predefined goggle geometry (or a portion thereof) 802. The process 800 may include one or more steps or processes 807 for aligning the face scan data 804, and optionally one or more data optimization and/or validation steps or processes 813. The process 800 may be implemented by one or more processors (e.g., processor 60) of the goggle customization system (e.g., computing device 30). The imported face scan data 804 is aligned via process 800 to the predefined goggle geometry such that the predefined goggle geometry is positioned, within the modeling environment (e.g., as visualized in FIG. 7B), relative to the user's face (which is represented by the face scan data) where the goggle would typically be positioned when worn. The process 800 provides, as output, the aligned face data 820, which includes at least a portion of the face scan data (i.e. 3D data of the user's face) aligned to the appropriate relative position in relation to the predefined goggle geometry, and optionally trimmed.

In some embodiments, the imported face scan data 804 may not be optimally oriented within the modeling environment relative to the predefined geometry. In such embodiments, upon receiving the user-specific data 805 as shown in the example in FIG. 8, the processor may perform a global alignment step/process (block 808). For example, the user's head (as represented by the face scan data 804) may be oriented relative to a reference coordinate frame (e.g., of the predefined goggle geometry or of the 3D modeling file created by the modeling environment upon importing of the face scan data). In some embodiments, orienting of the head at block 808 involves aligning the pupil landmarks (or other suitable landmark(s)) to a predefined position relative to the reference coordinate frame. For example, the point mid-way between the two pupil landmarks is positioned at a predetermined spatial coordinate (e.g., at x=0, y=0, and z=5mm relative to the reference coordinate frame). In other embodiments, a different global alignment of the imported user-specific data 805 may be performed to position the head in close proximity and suitable orientation to the goggle frame (e.g., as represented by the predefined geometry 802). In other embodiments, the global alignment may be performed by aligning other portions of the two imported data sets (e.g., the face scan data 804 and the predefined geometry 802) to one another. Also, while described herein as adjusting the position of the face scan data to align it to a global reference frame and/or the predefined geometry, in other embodiments, the predefined goggle geometry may additionally or alternatively be positionally adjusted to align it with the face scan data.

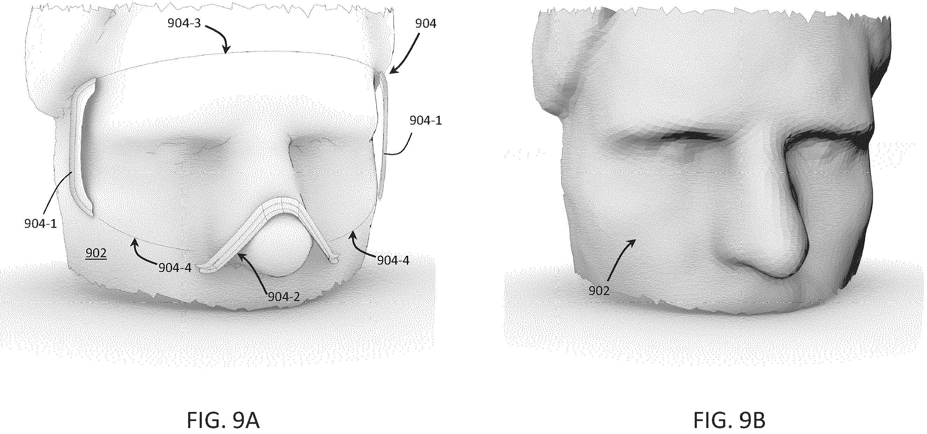

The alignment process may further involve the identifying of certain face landmarks (at block 810) and fine tuning the alignment (at block 812), such as by making fine adjustments through translation and/or rotation of the head relative to the limiting geometry. Certain face landmarks (e.g., the location of certain features of the user's face such as the forehead, temple, cheeks/check bones, nose bridge and nostrils) may be used in subsequent steps of the customization process and these landmarks may be identified at this stage, e.g., prior to trimming and/or adjusting the coarseness of the mesh. The fine tuning of the alignment (at block 812) may involve smaller positional adjustments as compared to the global alignment (e.g., the orienting of the face scan) via which the head (as represented by the face scan data) is positioned as close as possible, but without interference with (or intersecting) the limiting geometry. The term limiting geometry refers to a portion of the predefined goggle geometry, or to geometry defined based on the predefined goggle geometry, that is used during the alignment process. For example, and referring now also to FIG. 9A which shows an example visualization of face scan data 902 and limiting geometry 904, the limiting geometry 904 may include a portion of the geometry of any outriggers 904-1, if present in the particular selected goggle style, such as one or more curves or surfaces on the user-facing side of the outrigger(s). The limiting geometry 904-1 may set the lateral boundaries for the position of the user's head (as represented by the face scan data 902). The limiting geometry 904 may include a portion of the geometry of the nose piece 904-2 if present in the selected goggle, such as one or more curves or surfaces on the user-facing side of the nose piece. The limiting geometry 904 may include one or more curves (e.g. upper curve 904-3 and lower curves 904-4, representing the user-proximate edges of the vent foam(s)). The limiting geometries 904-2 through 904-4 may set vertical and rotational boundaries for the positioning of the user's head (as represented by the face scan data 902) in relation to the goggle. To complete the alignment process 807, the processor may adjust the position (e.g., translate and/or rotate) of the user's head iteratively and incrementally within the modeling environment until the head is positioned as close as possible to the limiting geometry, or within predefined thresholds, without intersecting the liming geometry.

In some embodiments, the process 800 may include additional, optional steps 813 that may improve the performance of the customization process. For example, the face scan data may be trimmed (see e.g., block 814) to remove portions of the face scan data (e.g., above the forehead and/or below the nose, as shown in FIG. 9A), thereby reducing the size of the dataset to be manipulated and thus improving computational efficiency. The trimming of the face scan data may be controlled in various ways, e.g., by reference to the face landmark data (e.g., trimming above the forehead and below a mid-point of the cheeks) and/or by reference to the limiting geometry. In some embodiments, multiple trimming steps may be performed. For example, a global trim step, which may be performed prior to alignment, may remove peripheral face scan data farther removed from the region of interest (e.g., the area including the eyes and around the eyes), and a second trim step, which may be performed after alignment, may remove additional face scan data to within a predefined thresholds (e.g., from the limiting geometry). In some embodiments, the trimmed dataset of the face scan data 902 may be resized, such as by changing (e.g., increasing) the coarseness of the mesh (as shown, for example in FIG. 9B), which may further reduce the processing burden and increase the computational speed of downstream processes (e.g., the face flange construction). In some embodiments, the mesh type of the trimmed/aligned face scan data may be modified, for example converting a triangular mesh to a mesh of other polygon shape (e.g., rectangular mesh). In some embodiments, a geometry validation check may be performed (e.g., at block 816) to assess the quality of the positioning of the face scan relative to the goggle. For example, the validation check may confirm proximity of the face scan data to fit markers, which may be part of the predefined goggle geometry. The validation check may also involve symmetry analysis, which may inform downstream steps of the customization process. Symmetry analysis may be performed by taking sections at different elevations and comparing the left and right sides of the face scan data along each section, or through proximity analysis between the aligned face scan data and the predefined goggle geometry. In some embodiments, failing to meet certain symmetry thresholds may indicate an increased risk for pool custom-fit output and thus the customization process may be terminated, debugged/checked manually and/or re-started (e.g., with the same or newly obtained user data).

After the datasets (e.g., the first dataset comprising the user/face data and the second dataset comprising the predefined goggle geometry) are properly aligned relative to one another (e.g., to mimic the position of the goggle on the user's face when worn), the custom goggle geometry is created. Depending on the particular goggle model selected by the user, the particulars of the portions of goggle geometry that is customized may vary in different embodiments. For example, some goggles may not utilize a face foam and thus the face foam geometry creation steps may be omitted. In some embodiments, the configuration of the vent flanges may differ from the specific example described below, and thus the steps for creating those portions of the custom geometry may be different.

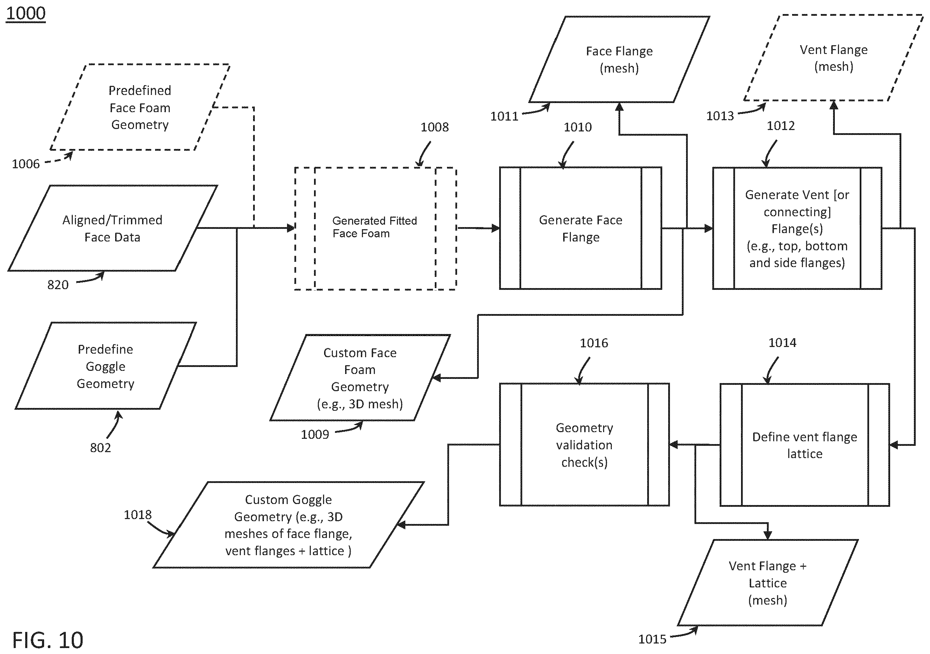

FIG. 10 shows a flow diagram of an example process 1000, which may be implemented by a processor 60 of the computing system 30 to create custom goggle geometry based on the user-specific face scan data. One or more of the blocks of process 1000 may be used to implement block 611 of the process 600. In some embodiments, one or more of the blocks of process 1000 may be omitted, combined or substituted with process blocks. For example, the step of generating fitted face foam at block 1008 is optionally, and may be omitted in embodiments in which the selected goggle does not have a face foam, or in embodiments in which face flange geometry is based on face scan data. In some embodiments, the face flange may be generated, similarly to the creation of the face foam geometry, such that it fits against the user's face.

The process 1000 is configured to create custom goggle geometry based on the user-specific face scan data (e.g., the aligned/trimmed face data 820 as generated by the alignment process 800), and further based on the predefined geometry (e.g., predefined geometry of the lens-side portion 219 of the goggle frame 210). The creation of custom goggle geometry via process 1000 may include the steps of defining or creating, e.g., within the 3D modeling environment, geometry for multiple portions of the goggle frame, for example the face flange, which is the portion of the goggle frame which conforms to the user's face when worn, and the vent flanges that connect the face flange to the predefined geometry of the front (or lens-side) portion of the goggle frame. In some embodiments, the process 1000 may include the steps of generating face flange geometry (at block 1010) based, in part, on 3D face scan data (e.g., aligned/trimmed face data 820), generating geometry for the one or more vent flanges (at block 1012) to connect the face flange to the predefined goggle geometry (e.g., the lens-side portion of the goggle frame), and defining a lattice for one or more of the vent flanges (at block 1014). In some embodiments, for example, where fitted face foam geometry is optionally generated, the process 1000 may include the steps of generating fitted face foam geometry (at block 1008), generating face flange geometry (at block 1010) based, in part, on the fitted face foam geometry, generating geometry for the one or more vent flanges (at block 1012) to connect the face flange to the predefined goggle geometry (e.g., the lens-side portion of the goggle frame), and defining a lattice for one or more of the vent flanges (at block 1014). As previously noted, geometry validation check(s) may be performed intermittently (e.g., at each step or upon completion of the custom goggle geometry creation) to ensure the quality and/or producibility of the custom-generated goggle geometry.

The process 1000 begins with the generating of fitted face foam geometry, e.g., in the form of a 3D mesh of the face foam shaped to fit against the user's face (i.e. against the face data 820). In some embodiments, the face foam is defined as a custom component and thus its geometry is defined from the face scan data, such as by offsetting a portion of the face data 820 a certain distance from the user's face. The offset defines the face foam in its fitted form (i.e. conformal to the user's face), which is then flattened to produce geometry (e.g., a 3D mesh defining the boundaries of a flat cut foam piece for purposes of production. The face foam offset distance may be pre-defined based on the compressed thickness of typical face foams, or it may be based on user-selected foam thickness. The fitted face foam geometry may then be used, at block 1010, to generate the face flange, such as by similarly offsetting the face foam geometry by a predetermined distance (i.e. corresponding to the desired thickness of the face flange 217). In embodiments in which custom face foam geometry is created (e.g., may be based on face flange geometry), the custom face foam geometry may be output, as a flattened 3D mesh 1009 for subsequent use (e.g., by a die cutter, laser cutter or other type of suitable foam cutting machine) to produce the physical custom face foam component.

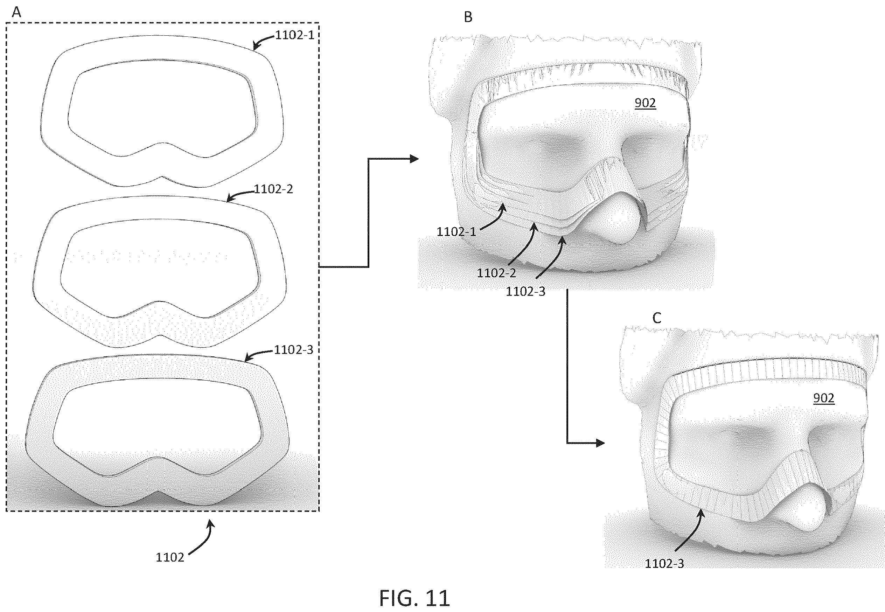

In some embodiments, the fitted face foam geometry is optionally generated (at block 1008) based, in part, on predefined face foam geometry 1006. Referring now also to FIG. 11, in some such embodiments, a plurality of predefined face foam geometries (e.g., flat 3D meshes of face foams 1102 of predetermined sizes) may be accessed (e.g., imported from storage 64 into the modeling environment) by processor 60. To generate the fitted face foam for the custom goggle at block 1008, the processor 60 may select 1008 one of the plurality of predefined face foam geometries, as shown in panel A of FIG. 11, by fitting each of the plurality of predefined face foam geometries (e.g., virtually fitting a first face foam mesh 1102-1, a second face foam mesh 1102-2 and/or a third face foam mesh 1102-3) to the face data 820 (e.g., to the mesh 902), as shown in panel B of FIG. 11, to determine the best fitting face foam mesh, as shown in panel C of FIG. 11. In some embodiments, each of the plurality of predefined face foam geometries is virtually fitted to the face data 820 using computer modeling techniques. In some embodiments, the processor 60 may perform the fitting concurrently, e.g., within the same modeling environment and may compare each of the fitted face foams to the others to determine the best fit. In such embodiments, the size of the selected face foam is stored for subsequent use (e.g., for generating the goggle frame markings at block 618, as will be further described).

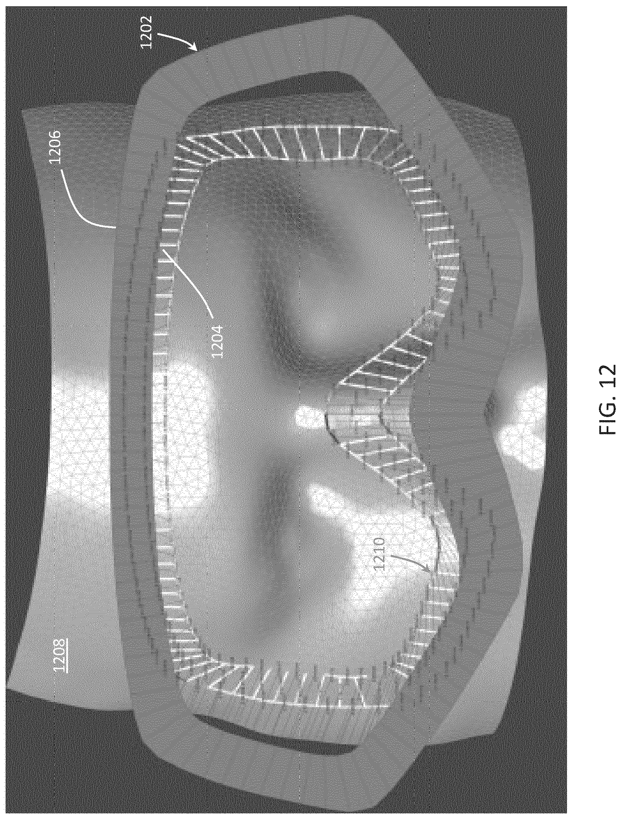

When using face foams of predetermined geometry, the flat 3D face foam mesh is fitted to the face scan mesh through a process, which may be iterative and/or incremental in some embodiments. The fitting process may involve aligning certain portion of the mesh to face fitting geometry, pulling the mesh to the face scan data (e.g., in some cases in a single adjustment, in some cases by incrementally adjusting the coordinates of vertices of the face foam mesh to coordinates of the face scan mesh vertices), and extending mesh edges as needed during this process, e.g., to match the contour of the face scan. The face fitting geometry may include one or more curves and/or vertices (e.g., along the forehead and/or at the nose), which may be used for initial alignment of the face foam mesh to the face data. In some embodiments, the steps of the fitting process are performed in different order or steps are substituted or combined. For example, in one embodiments, the mesh may be pulled (e.g., positionally adjusted) to the face scan data and mesh edges adjusted (e.g., shortened or extended) and the alignment relative to face fitting geometry may then be check as part of a validation process. Regardless of the specific process used to fit the mesh, the quality of the fit of the mesh may be determined using one or more fit criteria. For example, distortion analysis which measure the amount of distortion of individual or groups of mesh elements, may be used to determine if fitting the face foam to the face results in unacceptable amount (e.g., greater than 5%) distortion of the face foam mesh. FIG. 12 shows an example illustration of distortion analysis that may be performed as part of the face foam fitting process. In FIG. 12, the blank (or flat) face foam geometry 1202 (also referred to as flat mesh), which includes an inner boundary 1204 and outer boundary 1206 of a foam piece shaped to encircle a user's eyes, is shown in the same modeling space with a face data set (e.g., trimmed and aligned face scan of a user's face 1208). The corresponding fitted geometry (or fitted mesh) 1210 of the face foam is also visualized, together with the results of the distortion analysis, which may be visualized numerically and/or visually in the form of distortion vectors and/or color coding. Predefined face foam geometries that result in distortion above a predefined threshold, which may be based upon the foam material to be used in the production of the goggle, in order to obtain a fit may be deemed unsuitable for the particular user-specific face geometry. This process may be iterated with multiple (e.g., 2, 3 or in some cases more) predefined face foam geometries, to select the best fit and consequently the face foam size that is most suitable for the user's particular face shape. Certain other criteria may be employed, additionally or alternatively to distortion analysis, to perform the fit/selection process, such to verify the quality of the fit. For example, such criteria may include corner fit criteria (e.g., measuring the discrepancy between geometric alignment of the corners of the face foam and face data meshes against a set threshold/criteria). The fit criteria may additionally or alternatively include outer and/or inner boundary criteria, e.g., requirement that the outer and inner boundaries 1206 and 1204 of the foam mesh can be fitted to the contour of the face data mesh without excessive (e.g., more than 5%) distortion at the outer boundary and without substantially any distortion at the inner boundary, which can be measures as the delta in the segment lengths of the flat mesh 1202 vs. the fitted mesh 1210. The fit may additionally or alternatively be assessed using a symmetry criteria (e.g., a requirement that the left and right portions of the fitted mesh 1202 satisfy a preset symmetry threshold).

In some embodiments, whether using a predefined face foam geometry or whether creating a custom face foam geometry (e.g., through offsetting a portion of the face data), the face foam geometry at this stage of the process may define only a surface rather than a volumetric body, for example a mid-plane of the face foam or either of the two major, boundary surfaces of the face foam, i.e. the face-contacting surface or the gluing surface of the foam piece. In some such instances, particularly when creating a custom face foam piece for production, a thickness may be applied to the flattened custom face foam geometry to define a volume, and optionally the boundary edges of the volume may be filleted (to remove any sharp edges contacting the user's face), to produce the custom face foam geometry output at block 1009.

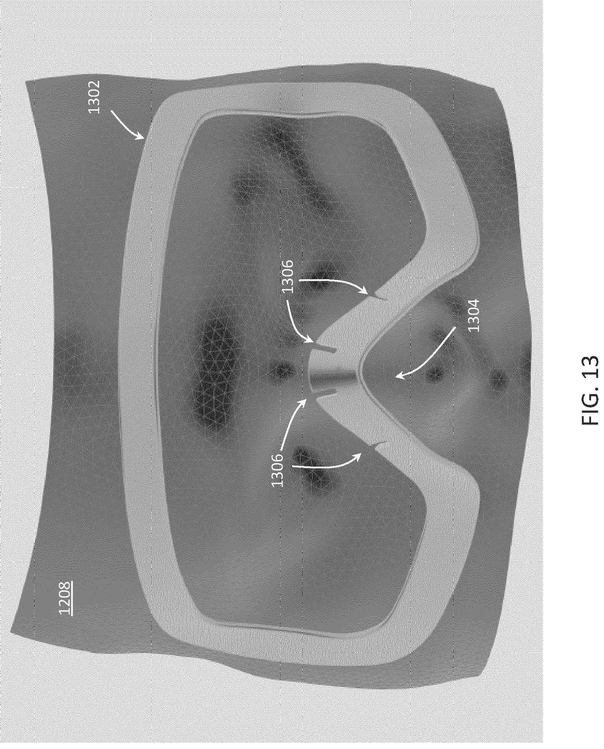

Continuing with block 1010, the process 1000 may then involve creating the face flange geometry based on the fitted face foam geometry created at block 1008. The face flange may be created by offsetting the face foam geometry, applying a thickness to the offset geometry, and then optionally filleting inner and outer boundary edges of the volume defined in the preceding step. A visualization of an example resulting face flange geometry 1302 is shown in FIG. 13. As also shown in FIG. 13, the process 1010 may additionally include defining one or more cuts 1306 at the nose region 1304 of the face flange 1302. In the illustrated example, the face flange includes 4 nose cuts, generally symmetrically distributed on the opposite side of the nose region. In other embodiments, fewer (e.g., 2 or 3) or greater number (e.g., 6, 7, etc.) may be used, which may depend upon the particular selected goggle model, the width of the flange, and in some cases user-specific factors, such as width and/or symmetry of the user's nose. When defining the nose cuts in the model, the nose cuts may be performed such that they meet certain criteria, for example being substantially vertically oriented in a front view of the face flange and being applied normal to the face flange surface.

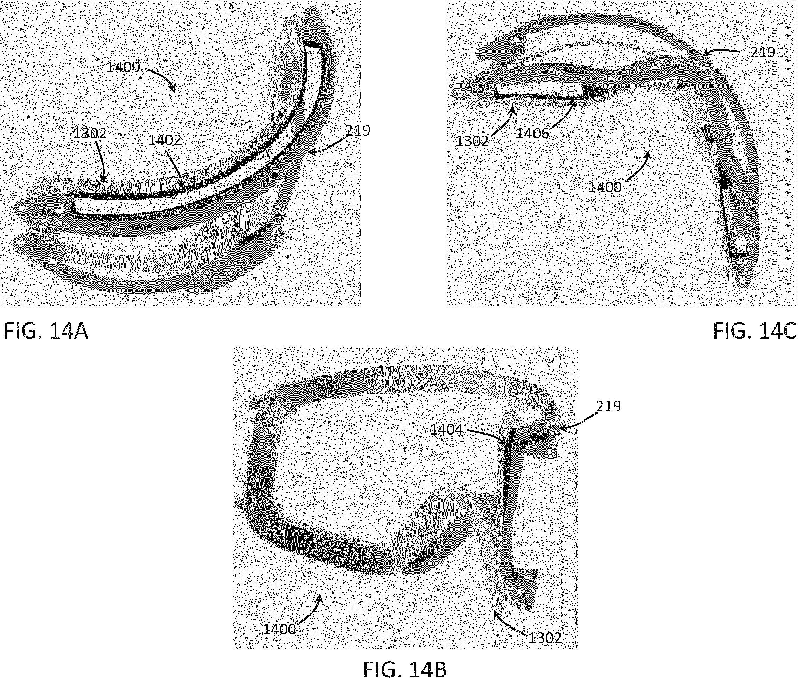

Returning back to the process 1000 of FIG. 10, and now also to FIGS. 14A-C, vent flanges are created to connect the face flange 1302 to the predetermined lens-side portion 219 of the goggle. FIGS. 14A-C shows top isometric, side isometric, and bottom isometric views of a portion of a goggle frame as modeled (e.g., in CAD), illustrating at least portions of the vent flanges of the goggle. A typical goggle 1400 includes a top vent flange 1402, two side vent flanges 1404 (only one side shown in FIG. 14B), and a bottom vent flange 1406. Thus, in step 1012, the geometry for the vent flanges of the goggle is defined using the face flange created at block 1010, the predefined goggle geometry (of the lens-side portion thereof) and geometry definition criteria as further described. To create the top vent flange 1402, the outer (or lens-facing) surface 1403 of the face flange 1302 may be trimmed to define the minor surface of the top vent flange 1409, which is then offset or lofted to connect it with the inner (or user-facing) surface 1405 of the predefined geometry (e.g., the lens-side portion 219 of the frame). The offset or lofting defines the major (or span) dimension of the top vent flange, creating generally a 3D volume. The trimming and offsetting (or lofting) processes are configured to ensure adherence with glue dam dimensions, such as to accommodate and enable securing of the vent foam, to be added during goggle assembly, within the recess defined by the vent flange between the face flange and lens-side portion 219 of the frame. Similar process is used to define the side and bottom vent flanges. Vent foam may also be attached at the bottom vent flange thus the geometry definition process for the bottom vent flange also similarly refers to glue dam criteria. In some embodiments, the order in which the vent flanges are created may be different, for example, creating the side flanges first, then the top and/or bottom vent flanges. In some embodiments, the geometry definition of the vent flanges may iteratively refine geometry previously created for one of the flanges (e.g., the top flange) after creating the geometry for an adjacent flange (e.g., the side flange(s)). Any other suitable geometry definition sequences may be used here to create the vent flange geometry. For example, the top and/or bottom vent flange geometries may be defined as picture-frame geometries, such as through a sequence of trimming and offsetting steps to define the boundary of the solid, leaving the interior of the vent flange mostly unfilled.

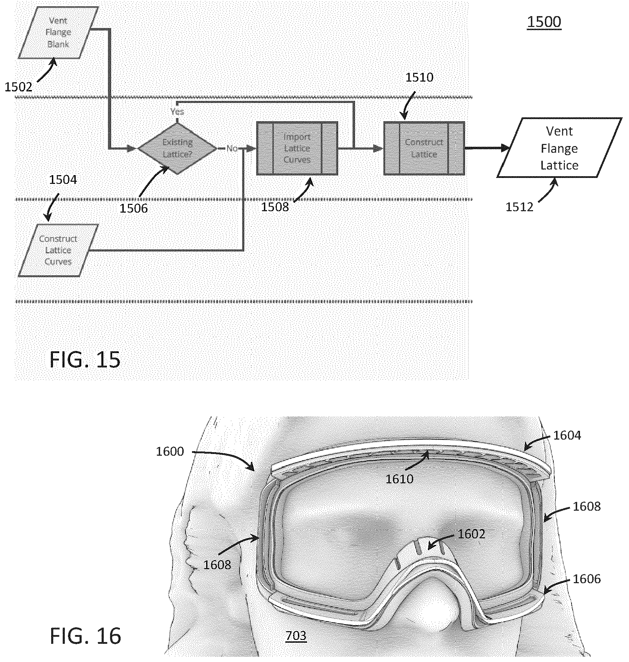

Next a vent flange lattice is defined over the span (or major) dimension of the tope and/or bottom vent flanges (e.g., at block 1014 of process 1000). The vent flange lattice may be defined in various ways, for example from existing geometry, in some cases additionally or alternatively based on user-selected options and/or optimization analysis. FIG. 15 shows an example process 1500 for creating a vent flange lattice 1512. The process 1500 may use as input the vent flange blank 1502, which may be the geometry of the 3D volume of the vent flange defined at block 1014 of process 1000. Additionally, and optionally if existing lattice geometry is to be used (see block 1506), the process 1500 receives the existing geometry (e.g., curve geometry defining the placement and contours of one or more members of the lattice structure. If an existing lattice geometry will not be used, the process 1500 constructs the lattice geometry (e.g., curves) within the same or external modeling environment (block 1504), and the curves are then imported into the same modeling space with the vent flange blank (see block 1508) such that the curves can them be used to guide the creation of the lattice structure of the custom vent flange (at block 1510). The process 1500 may output the vent lattice geometry 1512 (e.g., in the form of a mesh or other suitable geometry definition). FIG. 16 shows a visualization of the geometry portions created by the process so far (e.g. the custom-fit portion of the goggle frame) overlaid onto a visualization of the user's face (e.g., face scan data 703). Specifically, shown in the example in FIG. 16 are the face flange 1602, the top vent flange 1604 with it lattice structure 1610, as well as the side vent flanges 1608 and the bottom vent flange 1606.

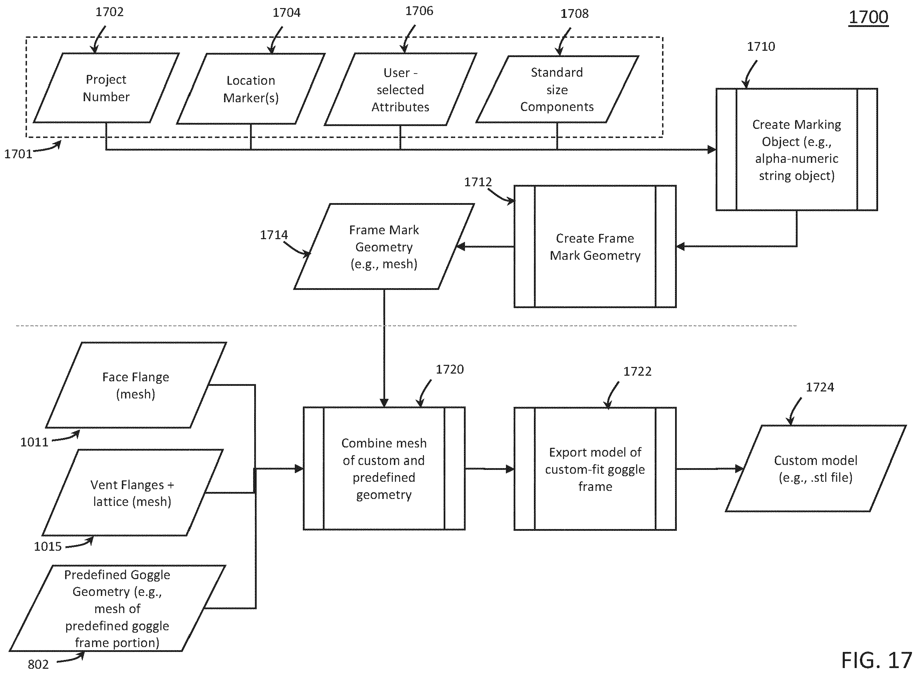

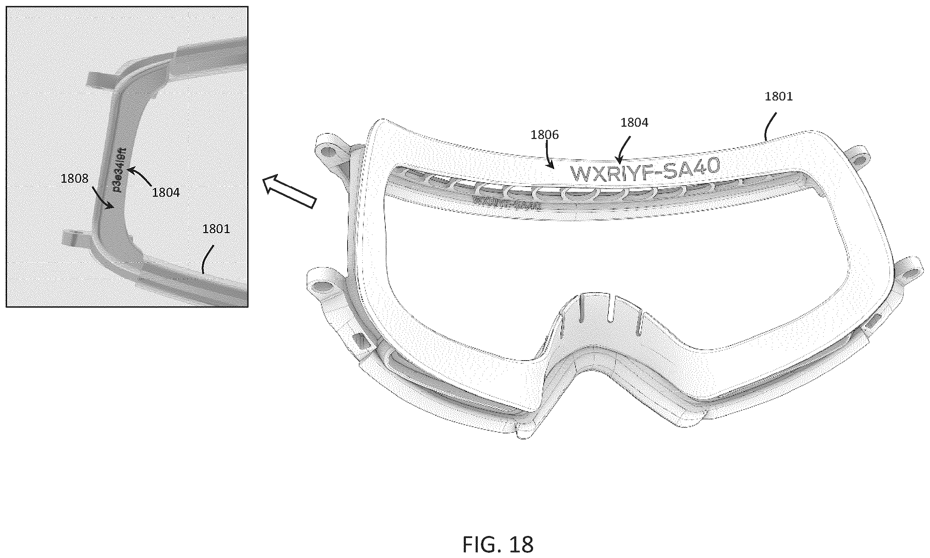

Referring now to FIGS. 17 and 18, an example process 1700 for goggle marking and data exporting will be further described. One or more of the blocks of process 1700 may be used implement process blocks (e.g., one or more of the blocks of 618-624) of the process 600 in FIG. 6. As shown in FIG. 17, the goggle marking process may receive various information as input 1701. For example, the inputs 1701 may include the project number 1702, which may be (or may be generated based on) the user's unique identifier created for and used in the goggle selection process (e.g., via the user app) describe above. Additionally or alternatively, information about user-selected attributes of the goggle (see block 1706), such as color, style or size of certain goggle components to be added to the goggle frame during assembly (e.g., outriggers, straps, nose piece, vent foams, etc.) may be provided and encoded into the product marking. Similarly, sizes or other identifiers (e.g., SKUs) of any standard size components (block 1708) that may be available in multiple standard sizes, may be encoded into the product marking. Location marker(s) (block 1704) which may specify the one or more locations on the goggle frame for placement of the product markings may also be provided. User-provided information may be included as input for the product marking, such as the user's name, user's initials, or other user-provided information. Any suitable combination including some or all of these inputs 1701 is received, at block 1710, which then creates the marking object based on the inputs.

The marking object may be an alpha numeric string, see e.g., markings 1802 and 1804). In some embodiments, multiple markings may be created and placed at different locations of the goggle frame 1801 to provide different information to different users, e.g., to an intermediate user who may be assembling the final custom goggle, and to and end user of the assembled custom goggle. For example, a first marking 1804 that conveys information to an intermediate user, such as the job number and/or about components to be assembled to the goggle frame (e.g., standard size components) may be provided at a first location 1806 of the goggle frame 1801. In some cases, the firs marking may be at a location 1806 that is ultimately concealed or covered (e.g., along the gluing surface of the face flange). Another marking 1804 may alternatively or additionally be provided at a second location 1808, which may not be concealed by the final goggle assembly. The marking 1804 may convey information to or be otherwise relevant to the end user (e.g., as a uniquely manufacturer's identifier of the custom goggle created specifically for this end user).

Referring back to process 1700, once the marking objects(s) have been defined, the frame marking geometry, which applies the marking object(s) to the appropriate locations on the goggle frame, is created at block 1712 and the frame mark geometry 1714 may be output (e.g., saved). To export the custom goggle (e.g., at block 624 of process 600), the processor obtains the various custom created geometries (e.g., the face flange 1011, the vent flanges and lattice 1015, the frame mark geometry 1714) and combines it, at block 1720, with the predefined goggle geometry, particularly the predefined portion (e.g., the lens-side portion 219) of the goggle frame to assemble the model of the custom-fit goggle frame, made specific for and based on a particular user's face scan data, which is then exported at block 1722 as a portable geometry file 1724. The portable geometry file contains the full definition of the goggle frame part to be 3D printed in a file format (e.g., an .stl file) compatible with a 3D printer of any suitable 3D printing technology. The model file 1724 may then be provided to the 3D printer whereby the custom goggle frame is printed and then assembled (e.g., by adding one or more standard components such as outrigger, strap, nose piece, and latch components, if any) to produce the final, assembled custom goggle. Using the custom markings on each custom goggle created by this process, the manufacturer/assembler of the final goggle can communicate progress of the assembly and shipment of the custom goggle (e.g., via the user app) to end user, enhancing the overall user experience.

The technology described herein may be implemented as logical operations and/or modules in one or more systems. The logical operations may be implemented as a sequence of processor implemented steps executing in one or more computer systems and as interconnected machine or circuit modules within one or more computer systems. Likewise, the descriptions of various component modules may be provided in terms of operations executed or effected by the modules. The resulting implementation is a matter of choice, dependent on the performance requirements of the underlying system implementing the described technology. Accordingly, the logical operations making up the embodiments of the technology described herein are referred to variously as operations, steps, objects, or modules. Furthermore, it should be understood that logical operations may be performed in any order, unless explicitly claimed otherwise or a specific order is inherently necessitated by the claim language. In some implementations, articles of manufacture are provided as computer program products that cause the instantiation of operations on a computer system to implement the invention. One implementation of a computer program product provides a non-transitory computer program storage medium readable by a computer system and encoding a computer program. It should further be understood that the described technology may be employed in special purpose devices independent of a personal computer.

The above specification, examples and data provide a complete description of the structure and use of exemplary embodiments of the invention as defined in the claims. Although various embodiments of the claimed invention have been described above with a certain degree of particularity, or with reference to one or more individual embodiments, those skilled in the art could make numerous alterations to the disclosed embodiments. Other embodiments are therefore contemplated. It is intended that all matter contained in the above description and shown in the accompanying drawings shall be interpreted as illustrative only of particular embodiments and not limiting. Changes in detail or structure may be made without departing from the basic elements of the invention as defined in the following claims. The foregoing description has broad application. The discussion of any embodiment is meant only to be explanatory and is not intended to suggest that the scope of the disclosure, including the claims, is limited to these examples. In other words, while illustrative embodiments of the disclosure have been described in detail herein, the inventive concepts may be otherwise variously embodied and employed, and the appended claims are intended to be construed to include such variations, except as limited by the prior art.

The foregoing discussion has been presented for purposes of illustration and description and is not intended to limit the disclosure to the form or forms disclosed herein. For example, various features of the disclosure are grouped together in one or more aspects, embodiments, or configurations for the purpose of streamlining the disclosure. However, various features of the certain aspects, embodiments, or configurations of the disclosure may be combined in alternate aspects, embodiments, or configurations.

The invention is defined by the independent claims and preferred features are set out in the dependent claims. All directional references (e.g., proximal, distal, upper, lower, upward, downward, left, right, lateral, longitudinal, front, back, top, bottom, above, below, vertical, horizontal, radial, axial, clockwise, and counterclockwise) are only used for identification purposes to aid the reader's understanding of the present disclosure, and do not create limitations, particularly as to the position, orientation, or use. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority, but are used to distinguish one feature from another. The drawings are for purposes of illustration only and the dimensions, positions, order and relative sizes reflected in the drawings attached hereto may vary.

## Claims

1. A computer-implemented method for customizing a goggle (11), comprising:
receiving (410) three-dimensional (3D) face scan data (602) of at least a portion of a face of a user, wherein the 3D face scan data comprises data for a 3D representation of at least a portion of the face of the user;
generating (420) a custom 3D model of a goggle frame based, at least in part, on the 3D face scan data, wherein the generating the custom 3D model of the goggle frame includes:
defining (612) geometry of a face flange portion of the goggle frame based, at least in part, on the 3D face scan data; and
defining (614) geometry of one or more vent flange portions of the goggle frame which connect the face flange portion to a geometry of a lens-side portion of the goggle frame to generate the custom 3D model of the goggle frame;
creating fitted face foam geometry, and wherein defining geometry of the face flange portion of the goggle frame is based, at least in part, on the fitted face foam geometry; and
outputting (430) a 3D-printer compatible file (606) of the custom 3D model of the goggle frame.

2. The method of claim 1 wherein the geometry of the lens-side goggle of the goggle frame comprises predetermined geometry of a lens-side portion of the goggle frame.

3. The method of claim 1 wherein the geometry of the lens-side portion of the goggle frame comprises a plurality of predetermined geometries for the lens-side portion of the goggle frame.

4. The method of claim 1 further comprising selecting the geometry of the lens-side portion of the goggle frame from a plurality of predetermined geometries of the lens-side portion of the goggle frame.

5. The method of claim 1 further comprising receiving face landmark data together with the 3D face scan data.

6. The method of claim 5 further comprising importing the 3D face scan data into a computer-aided design (CAD) program together with an existing CAD model containing at least the geometry of the lens-side portion of the goggle frame.

7. The method of claim 6 wherein the data for the 3D representation of at least a portion of the face of the user includes a 3D mesh representation of at least a portion of the face of the user, and the method further comprising aligning the 3D mesh of the 3D face scan data to the existing CAD model using the face landmark data.

8. The method of claim 7 wherein the aligning of the 3D mesh to the existing CAD model comprises aligning pupil center landmark points to predetermined horizontal and vertical locations of the CAD model.

9. The method of claim 7 further comprising defining goggle limiting geometry based on one or more components of the existing CAD model and aligning the 3D mesh to the goggle limiting geometry.

10. The method of claim 9 further comprising trimming the 3D mesh of the 3D face scan data to obtain a partial 3D face mesh prior to aligning the 3D mesh to the goggle limiting geometry.

11. The method of claim 10 further comprising increasing a coarseness of the partial 3D mesh prior to aligning the 3D mesh to the goggle limiting geometry.

12. The method of claim 9 wherein the existing CAD model further comprises geometry for at least one of strap outriggers and a nose piece of the goggle frame, and wherein the goggle limiting geometry is further based on the geometry of the strap outriggers and/or the geometry of the nose piece.

13. The method of claim 1 wherein the 3D face scan data is obtained using a scanner.

14. The method of claim 13 wherein the 3D face scan data of at least a portion of the face of the user is obtained using the scanner as the face of the user is rotated through a range of angles relative to the scanner.

15. The method of claim 13 wherein the 3D face scan data of at least a portion of the face of the user is obtained using the scanner as the scanner is rotated through a range of angles relative to the face of the user.

16. The method of claim 1 wherein defining the geometry of the face flange portion of the goggle frame comprises:
offsetting the fitted face foam geometry by a predetermined amount to define a face foam attachment surface of the face flange portion; and
offsetting the face foam attachment surface in a direction away from the fitted face foam by a predetermined distance to define a thickness of the face flange portion.

17. The method of claim 16 wherein defining the geometry of the one or more vent flange portions of the goggle frame comprises defining one or more transverse surfaces that connect the lens-side portion of the goggle frame to a second surface of the face flange portion opposite the face foam attachment surface.

18. The method of claim 1 wherein defining the geometry of the face flange portion of the goggle frame comprises adjusting the face flange portion based on at least nostrils, temple, or combinations thereof of the face of the user.

## Patentansprüche

1. Computerimplementiertes Verfahren zum benutzerdefinierten Anpassen einer Brille (11), das Folgendes umfasst:
Empfangen (410) dreidimensionaler (3D) Gesichtscandaten (602) von zumindest einem Abschnitt eines Gesichts eines Benutzers, wobei die 3D-Gesichtscandaten Daten für eine 3D-Darstellung von zumindest einem Abschnitt des Gesichts des Benutzers umfassen;
Generieren (420) eines benutzerdefinierten 3D-Modells eines Brillenrahmens zumindest teilweise basierend auf den 3D-Gesichtscandaten, wobei das Generieren des benutzerdefinierten 3D-Modells des Brillenrahmens Folgendes einschließt:
Definieren (612) der Geometrie eines Gesichtsflanschabschnitts des Brillenrahmens zumindest teilweise basierend auf den 3D-Gesichtsscandaten; und
Definieren (614) einer Geometrie eines oder mehrerer Entlüftungsflanschabschnitte des Brillenrahmens, die den Gesichtsflanschabschnitt mit einer Geometrie eines linsenseitigen Abschnitts des Brillenrahmens verbinden, um das benutzerdefinierte 3D-Modell des Brillenrahmens zu generieren;
Generieren einer angepassten Gesichtsschaumgeometrie, und wobei Definieren der Geometrie des Gesichtsflanschabschnitts des Brillenrahmens zumindest teilweise auf der angepassten Gesichtsschaumgeometrie basiert; und
Ausgeben (430) einer mit einem 3D-Drucker kompatiblen Datei (606) des benutzerdefinierten 3D-Modells des Brillenrahmens.

2. Verfahren nach Anspruch 1, wobei die Geometrie der linsenseitigen Brille des Brillenrahmens eine vorbestimmte Geometrie eines linsenseitigen Abschnitts des Brillenrahmens umfasst.

3. Verfahren nach Anspruch 1, wobei die Geometrie des linsenseitigen Abschnitts des Brillenrahmens eine Vielzahl von vorbestimmten Geometrien für den linsenseitigen Abschnitt des Brillenrahmens umfasst.

4. Verfahren nach Anspruch 1, ferner umfassend Auswählen der Geometrie des linsenseitigen Abschnitts des Brillenrahmens aus einer Vielzahl von vorbestimmten Geometrien des linsenseitigen Abschnitts des Brillenrahmens.

5. Verfahren nach Anspruch 1, ferner umfassend Empfangen von Gesichtslandmarkendaten zusammen mit den 3D-Gesichtsscandaten.

6. Verfahren nach Anspruch 5, ferner umfassend Importieren der 3D-Gesichtsscandaten in ein computergestütztes Design(CAD)-Programm zusammen mit einem vorhandenen CAD-Modell, das zumindest die Geometrie des linsenseitigen Abschnitts des Brillenrahmens enthält.

7. Verfahren nach Anspruch 6, wobei die Daten für die 3D-Darstellung von zumindest einem Abschnitt des Gesichts des Benutzers eine 3D-Netzdarstellung von zumindest einem Abschnitt des Gesichts des Benutzers einschließen, und wobei das Verfahren ferner Ausrichten des 3D-Netzes der 3D-Gesichtscandaten an dem bestehenden CAD-Modell unter Verwendung der Gesichtslandmarkendaten umfasst.

8. Verfahren nach Anspruch 7, wobei das Ausrichten des 3D-Netzes an dem bestehenden CAD-Modell Ausrichten von Pupillenzentrumlandmarkenpunkten an vorbestimmten horizontalen und vertikalen Positionen des CAD-Modells umfasst.

9. Verfahren nach Anspruch 7, ferner umfassend Definieren einer Brillenbegrenzungsgeometrie basierend auf einer oder mehreren Komponenten des bestehenden CAD-Modells und Ausrichten des 3D-Netzes an der Brillenbegrenzungsgeometrie.

10. Verfahren nach Anspruch 9, ferner umfassend Zuschneiden des 3D-Netzes der 3D-Gesichtsscandaten, um ein partielles 3D-Gesichtsnetz vor dem Ausrichten des 3D-Netzes auf die Brillenbegrenzungsgeometrie zu erhalten.

11. Verfahren nach Anspruch 10, ferner umfassend Erhöhen einer Grobheit des partiellen 3D-Netzes vor Ausrichten des 3D-Netzes auf die Brillenbegrenzungsgeometrie.

12. Verfahren nach Anspruch 9, wobei das bestehende CAD-Modell ferner die Geometrie von zumindest einem von Bandaufhängung und einem Nasenstück des Brillenrahmens umfasst, und wobei die Brillenbegrenzungsgeometrie ferner auf der Geometrie der Bandaufhängung und/oder der Geometrie des Nasenstücks basiert.

13. Verfahren nach Anspruch 1, wobei die 3D-Gesichtsscandaten unter Verwendung eines Scanners erhalten werden.

14. Verfahren nach Anspruch 13, wobei die 3D-Gesichtsscandaten von zumindest einem Abschnitt des Gesichts des Benutzers unter Verwendung des Scanners erhalten werden, während das Gesicht des Benutzers um einen Bereich von Winkeln relativ zu dem Scanner gedreht wird.

15. Verfahren nach Anspruch 13, wobei die 3D-Gesichtsscandaten zumindest eines Abschnitts des Gesichts des Benutzers unter Verwendung des Scanners erhalten werden, während der Scanner um einen Bereich von Winkeln relativ zu dem Gesicht des Benutzers gedreht wird.

16. Verfahren nach Anspruch 1, wobei Definieren der Geometrie des Gesichtsflanschabschnitts des Brillenrahmens Folgendes umfasst:
Versetzen der angepassten Gesichtsschaumgeometrie um einen vorbestimmten Betrag, um eine Gesichtsschaumbefestigungsfläche des Gesichtsflanschabschnitts zu definieren; und
Versetzen der Gesichtsschaumbefestigungsfläche in eine Richtung weg von dem angepassten Gesichtsschaum um einen vorbestimmten Abstand, um eine Dicke des Gesichtsflanschabschnitts zu definieren.

17. Verfahren nach Anspruch 16, wobei Definieren der Geometrie des einen oder der mehreren Entlüftungsflanschabschnitte des Brillenrahmens Definieren einer oder mehrerer Querflächen umfasst, die den linsenseitigen Abschnitt des Brillenrahmens mit einer zweiten Oberfläche des Gesichtsflanschabschnitts gegenüber der Gesichtsschaumbefestigungsoberfläche verbinden.

18. Verfahren nach Anspruch 1, wobei Definieren der Geometrie des Gesichtsflanschabschnitts des Brillenrahmens Einstellen des Gesichtsflanschabschnitts basierend auf zumindest Nasenlöchern, Schläfe oder Kombinationen davon des Gesichts des Benutzers umfasst.

## Revendications

1. Procédé mis en œuvre par ordinateur pour personnaliser des lunettes de sécurité (11), comprenant :
la réception (410) de données de balayage de visage tridimensionnelles (3D) (602) d'au moins une partie d'un visage d'un utilisateur, dans lequel les données de balayage de visage 3D comprennent des données pour une représentation 3D d'au moins une partie du visage de l'utilisateur ;
la génération (420) d'un modèle 3D personnalisé d'une monture de lunettes de sécurité sur la base, au moins en partie, des données de balayage de visage 3D, dans lequel la génération du modèle 3D personnalisé de la monture de lunettes de sécurité inclut :
la définition (612) d'une géométrie d'une partie bride de visage de la monture de lunettes de sécurité sur la base, au moins en partie, des données de balayage de visage 3D ; et
la définition (614) d'une géométrie d'un ou de plusieurs parties brides de ventilation de la monture de lunettes de sécurité qui relient le partie bride de visage à une géométrie d'une partie côté verre de la monture de lunettes de sécurité pour générer le modèle 3D personnalisé de la monture de lunettes de sécurité ;
la création d'une géométrie de mousse de visage ajustée, et dans lequel la définition d'une géométrie de la partie bride de visage de la monture de lunettes de sécurité est sur la base, au moins en partie, de la géométrie de mousse de visage ajustée ; et
la sortie (430) d'un fichier compatible imprimante 3D (606) du modèle 3D personnalisé de la monture de lunettes de sécurité.

2. Procédé de la revendication 1, dans lequel la géométrie des lunettes de sécurité côté verre de la monture de lunettes de sécurité comprend une géométrie prédéterminée d'une partie côté verre de la monture de lunettes de sécurité.

3. Procédé de la revendication 1, dans lequel la géométrie de la partie côté verre de la monture de lunettes de sécurité comprend une pluralité de géométries prédéterminées pour la partie côté verre de la monture de lunettes de sécurité.

4. Procédé de la revendication 1, comprenant en outre la sélection de la géométrie de la partie côté verre de la monture de lunettes de sécurité parmi une pluralité de géométries prédéterminées de la partie côté verre de la monture de lunettes de sécurité.

5. Procédé de la revendication 1, comprenant en outre la réception de données de repères de visage conjointement avec les données de balayage de visage 3D.

6. Procédé de la revendication 5, comprenant en outre l'importation des données de balayage de visage 3D dans un programme de conception assisté par ordinateur (Computer-Aided Design, CAD) conjointement avec un modèle CAD existant contenant au moins la géométrie de la partie côté verre de la monture de lunettes de sécurité.

7. Procédé de la revendication 6, dans lequel les données pour la représentation 3D d'au moins une partie du visage de l'utilisateur incluent une représentation maille 3D d'au moins une partie du visage de l'utilisateur, et le procédé comprenant en outre l'alignement de la maille 3D des données de balayage de visage 3D avec le modèle CAD existant en utilisant les données de repères de visage.

8. Procédé de la revendication 7, dans lequel l'alignement de la maille 3D avec le modèle CAD existant comprend l'alignement de points de repères de centre de pupille avec des emplacements horizontaux et verticaux prédéterminés du modèle CAD.

9. Procédé de la revendication 7, comprenant en outre la définition d'une géométrie limitatrice de lunettes de sécurité sur la base d'un ou de plusieurs composants du modèle CAD existant et l'alignement de la maille 3D avec la géométrie limitatrice de lunettes de sécurité.

10. Procédé de la revendication 9, comprenant en outre la réduction de la maille 3D des données de balayage de visage 3D pour obtenir une maille de visage 3D partielle avant l'alignement de la maille 3D avec la géométrie limitatrice de lunettes de sécurité.

11. Procédé de la revendication 10, comprenant en outre l'augmentation d'une grossièreté de la maille 3D partielle avant l'alignement de la maille 3D avec la géométrie limitatrice de lunettes de sécurité.

12. Procédé de la revendication 9, dans lequel le modèle CAD existant comprend en outre une géométrie pour au moins un de portants de lanière et un pont de nez de la monture de lunettes de sécurité, et dans lequel la géométrie limitatrice de lunettes de sécurité est en outre basée sur la géométrie des portants de lanière et/ou la géométrie du pont de nez.

13. Procédé de la revendication 1, dans lequel les données de balayage de visage 3D sont obtenues en utilisant un dispositif de balayage.

14. Procédé de la revendication 13, dans lequel les données de balayage de visage 3D d'au moins une partie du visage de l'utilisateur sont obtenues en utilisant le dispositif de balayage quand le visage de l'utilisateur est mis en rotation à travers une plage d'angles relativement au dispositif de balayage.

15. Procédé de la revendication 13, dans lequel les données de balayage de visage 3D d'au moins une partie du visage de l'utilisateur sont obtenues en utilisant le dispositif de balayage quand le dispositif de balayage est mis en rotation à travers une plage d'angles relativement au visage de l'utilisateur.

16. Procédé de la revendication 1, dans lequel la définition de la géométrie de la partie bride de visage de la monture de lunettes de sécurité comprend :
le décalage de la géométrie de mousse de visage ajustée selon une quantité prédéterminée pour définir une surface d'attache de mousse de visage du partie bride de visage ; et
le décalage de la surface d'attache de mousse de visage dans une direction à l'opposé de la mousse de visage ajustée selon une distance prédéterminée pour définir une épaisseur de la partie bride de visage.

17. Procédé de la revendication 16, dans lequel la définition de la géométrie de l'une ou des plusieurs parties brides de ventilation de la monture de lunettes de sécurité comprend la définition d'une ou de plusieurs surfaces transversales qui relient la partie côté verre de la monture de lunettes de sécurité à une seconde surface de la partie bride de visage en face de la surface d'attache de mousse de visage.

18. Procédé de la revendication 1, dans lequel la définition de la géométrie de la partie bride de visage de la monture de lunettes de sécurité comprend l'ajustement de la partie bride de visage sur la base au moins de narines, d'une tempe, ou de combinaisons de celles-ci, du visage de l'utilisateur.
